# EUROPEAN PATENT SPECIFICATION

(11) **EP 3 302 522 B1**
(45) Date of publication and mention of the grant of the patent: **31.08.2022**
(21) Application number: 16744567.5
(22) Date of filing: 02.06.2016
(51) Int. Cl.: A61K 38/17, A61K 45/00, A61K 48/00, A61P 21/00

(54) **MEANS AND METHODS FOR TREATING FACIOSCAPULOHUMERAL MUSCULAR DYSTROPHY (FSHD).**
MITTEL UND VERFAHREN ZUR BEHANDLUNG VON FAZIOSKAPULOHUMERALER MUSKELDYSTROPHIE (FSHD)
MOYENS ET PROCÉDÉS POUR LE TRAITEMENT DE LA DYSTROPHIE FACIO-SCAPULO-HUMÉRALE

(30) Priority: 02.06.2015 EP 15170244
(43) Date of publication of application: 11.04.2018
(73) Proprietor: Academisch Ziekenhuis Leiden h.o.d.n. LUMC, 2333 ZA Leiden (NL); Fred Hutchinson Cancer Center, Seattle, WA 98109 (US); University of Rochester, Rochester, NY 14642 (US)
(72) Inventor: VAN DER MAAREL, Silvere Maria, 2333 ZA Leiden (NL); LEMMERS, Richard Joannes Leonardus Franciscus, 2333 ZA Leiden (NL); BALOG, Judit, 2333 ZA Leiden (NL); TAPSCOTT, Stephen Justice, 2333 ZA Leiden (NL); TAWIL, Alrabi, 2333 ZA Leiden (NL)
(74) Representative: V.O.
(86) International application number: PCT/NL2016/050396
(87) International publication number: WO 2016/195493

(56) References cited:
- WO-A2-2013/120038
- SILVRE M VAN DER MAAREL ET AL: "Facioscapulohumeral muscular dystrophy and DUX4: breaking the silence", TRENDS IN MOLECULAR MEDICINE, vol. 17, no. 5, 2011, pages 252-258, XP028206228, ISSN: 1471-4914, DOI: 10.1016/J.MOLMED.2011.01.001 [retrieved on 2011-01-05] cited in the application
- SACCONI SABRINA ET AL: "The FSHD2 Gene SMCHD1 Is a Modifier of Disease Severity in Families Affected by FSHD1", AMERICAN JOURNAL OF HUMAN GENETICS, vol. 93, no. 4, October 2013 (2013-10), pages 744-751, XP055218974,
- RICHARD J L F LEMMERS ET AL: "Digenic inheritance of an SMCHD1 mutation and an FSHD-permissive D4Z4 allele causes facioscapulohumeral muscular dystrophy type 2", NATURE GENETICS, vol. 44, no. 12, 11 November 2012 (2012-11-11), pages 1370-1374, XP055177823, ISSN: 1061-4036, DOI: 10.1038/ng.2454
- SACCONI SABRINA ET AL: "The FSHD2 Gene SMCHD1 Is a Modifier of Disease Severity in Families Affected by FSHD1", AMERICAN JOURNAL OF HUMAN GENETICS, vol. 93, no. 4, October 2013 (2013-10), pages 744-751, ISSN: 0002-9297(print)

## Description

### FIELD OF THE INVENTION

The present invention in general relates to the field of medicine and particularly to medicaments and therapies for the treatment of facioscapulohumeral muscular dystrophy (FSHD). More particularly the present invention relates to the use of a protein encoded by a structural maintenance of chromosomes flexible hinge domain containing 1 (*SMCHD1*) gene or means for regulating the activity thereof in a cell for treating FSHD.

### BACKGROUND OF THE INVENTION

As one of the most prevalent forms of muscular dystrophy, with recent studies in the Netherlands reporting that approximately 12 in 100,000 individuals are affected, and with no effective treatment being available, FSHD makes an interesting subject for development of therapeutic products and therapies. FSHD is genetically linked to the polymorphic D4Z4 macrosatellite repeat array at the subtelomeric region 4q35 on chromosome 4, which repeat array in healthy subjects consists of 8-100 units, each of the units being 3.3 kb in size and containing a copy of the *DUX4* gene. The *DUX4* gene, coding for the germline double homeobox transcription factor DUX4, is normally silenced in somatic cells by repeat mediated epigenetic repression.

Subjects suffering from FSHD however demonstrate an inadequate repeat mediated epigenetic repression of DUX4 in skeletal muscle cells. Because of a decompaction of the D4Z4 chromatin structure there is an elevated expression of DUX4 in those cells. Stable *DUX4* expression requires a specific genetic background of chromosome 4 that contains a polymorphic *DUX4*poly-adenylation signal (PAS) immediately distal to the D4Z4 repeat array. A D4Z4 chromatin decompaction on a *DUX4*-PAS containing chromosome leads to the FSHD-specific pattern of *DUX4* expression in cells, expressing relatively abundant amounts of DUX4 protein in some myonuclei in tissue culture. Expression of DUX4 protein is harmful to skeletal muscle since it modulates inflammatory pathways, and germline and early developmental pathways, which eventually may result in increased cell death. Hence subjects suffering from the disease typically in the second decade of life start to demonstrate a progressive and often asymmetrical weakness of facial and upper extremity muscles.

In at least 95% of FSHD individuals the inadequate *DUX4* repression can be attributed to a contraction of the D4Z4 repeat array to a size of 1-10 units. This more common form of FSHD is known as FSHD type 1 (FSHD1). In the remainder of individuals (<5%) the D4Z4 repeat arrays are in the normal size range. The majority of these individuals carry heterozygous mutations in the *SMCHD1* gene on chromosome 18. This less common form of FSHD is known as FSHD type 2 (FSHD2). Individuals with FSHD1 with a D4Z4 repeat array to a size of 1-10 units that also carry the *SMCHD1* gene with heterozygous mutations demonstrate increased FSHD disease severity. Such diseases are classified as FSHD1 in the present invention.

The *SMCHD1* gene codes for a chromatin repressor protein that binds to D4Z4. SMCHD1 is also known as SMC hinge domain-Containing Protein 1; KIAA0650; Structural Maintenance Of Chromosomes Flexible Hinge Domain-Containing Protein 1. External Ids are HGNC: 29090; Entrez Gene: 23347; Ensembl: ENSG00000101596; OMIM: 614982 and UniProtKB: A6NHR9.

Germline mutations in *SMCHD1* have been shown to result in reduced D4Z4 chromatin compaction and increased DUX4 expression. The precise role of SMCHD1 protein on the chromatin structure of particularly the D4Z4 repeat array remains to be determined, and further data on the epigenetic regulation of the D4Z4 repeat array are scarce. Extensive changes in the genome wide chromatin structure have been reported upon myogenic differentiation in mouse cells. It however proves a challenge to identify changes in D4Z4 chromatin regulation specific to differentiated muscle cells that might account for the susceptibility of this tissue to express DUX4 at higher levels than in myoblasts or many other tissues.

In somatic cells originating from healthy individuals, the D4Z4 repeat array is characterized by high, but inhomogeneous, levels of CpG methylation and histone 3 lysine 9 trimethylation (H3K9me3). Proliferating FSHD somatic cell cultures demonstrate a reduction of both CpG methylation and H3K9me3 epigenetic markers at D4Z4 together with reduced cohesin and heterochromatin protein 1 gamma (HP1γ) levels, presumably facilitating sporadic activation of *DUX4.* SMCHD1 protein levels at D4Z4 decline during muscle cell differentiation and correlate with increased Polycomb repressive complex 2 (PRC2) and H3K27me3 levels at D4Z4 in FSHD1 and FSHD2 muscle cell cultures. *SMCHD1* knockdown in control myotubes leads to DUX4 activation and this also correlates with increased Polycomb repressive complex 2 (PRC2) and H3K27me3 levels at D4Z4. Accordingly, in FSHD1 and FSHD2 the natural decline in SMCHD1 protein levels observed during normal muscle cell differentiation renders these skeletal muscle cells sensitive for DUX4 expression.

Current approaches for therapeutic intervention in FSHD are mostly focused on DUX4 by targeting DUX4 mRNA or blocking activity of DUX4 protein. The processes induced by DUX4 expression that lead to the pathology are also investigated as a possible way to treat the disease. A more preventive approach however is to reduce DUX4 expression, for example by enhancing the epigenetic repression of the D4Z4 repeat. For this there is a need to develop methods and compositions that enable reduction of DUX4 expression in order to treat FSHD, both type 1 and type 2. The present invention thus aims among others to provide such a method and composition, and in particular aims to provide a method and composition for treating FSHD by reducing DUX4 expression in muscular cells by restoring and/or enhancing epigenetic repression of D4Z4.

### SUMMARY OF THE INVENTION

Epigenetics refers among others to functionally relevant changes to the genome that do not involve a change in the nucleotide sequence. Examples of mechanisms that produce such changes are DNA methylation and histone modification, each of which alters how genes are expressed without altering the underlying DNA sequence. Gene expression can be controlled through the action of repressor proteins that attach to silencer regions of the DNA. These epigenetic changes may last through cell divisions for the duration of the cell's life, and may also last for multiple generations even though they do not involve changes in the underlying DNA sequence of the organism; instead, non-genetic factors cause the organism's genes to behave (or "express themselves") differently. Various epigenetic mechanisms have been identified and likely more remain to be discovered.

It has been found that that inactivating mutations in the SMCHD1 gene are associated with FSHD, in particular with FSHD2. In the present invention it was surprisingly found that overexpression of SMCHD1 results in DUX4 silencing in FSHD1 and FSHD2 myotubes. This demonstrates that the reduction in DUX4 repression in FSHD is surprisingly reversible. Epigenetic phenomena are dynamic, but not always automatically reversible. MeCP2, for instance, is a protein that binds to forms of DNA that has been methylated. The MeCP2 protein then interacts with other proteins to form a complex that turns off the target gene. Mammalian methylation occurs in particular in the DNA sequence "CpG". Many genes have so-called CpG islands, which frequently occur near the beginning of the gene. MeCP2 does not bind to these islands in most cases, as they are not methylated. The expression of a number of genes may be regulated through methylation of their CpG island, and MeCP2 may play a role in a subset of these. One of the functions of MeCP2 is thus the silencing of methylated promoters. The phenotype of a genetic depletion of MeCP2 through an inactivating mutation is not readily reversible by providing excess MeCP2 protein. DNMT3B is another protein involved in epigenetic phenomena. DNMT3B is DNA (cytosine-5-)-methyltransferase 3 beta, is a protein associated with immunodeficiency, centromere instability and facial anomalies (ICF) syndrome. It is also known as ICF1; M.HsaIIIB; ICF; DNA Methyltransferase HsaIIIB; DNA (Cytosine-5)-Methyltransferase 3B; DNA MTase HsaIIIB; Dnmt3b; and EC 2.1.1.37. External Ids are: HGNC: 2979; Entrez Gene: 1789; Ensembl: ENSG00000088305; OMIM: 602900; UniProtKB: Q9UBC3. CpG methylation is an epigenetic modification that is important for embryonic development, imprinting, and X-chromosome inactivation. Studies in mice have demonstrated that DNA methylation is required for mammalian development. DNMT3B encodes a DNA methyltransferase which is thought to function in de novo methylation, rather than maintenance methylation. The protein localizes primarily to the nucleus and its expression is developmentally regulated. Mutations in this gene cause the autosomal recessive immunodeficiency-centromeric instability-facial anomalies (ICF) syndrome type 1. In the present invention it was found that DNMT3B is an FSHD2 gene. Autosomal dominant mutations in the DNMT3B gene are associated with FSHD2 individuals that lack a mutation in SMCHD1. It is a disease modifier for FSHD1 and is associated with hypomethylation of the D4Z4 repeat and expression of DUX4. It was found that ectopic expression of DNMT3B in FSHD muscle cells did not reverse an FSHD phenotype. For instance, DUX4 repression was not increased by providing ectopic DNMT3B.

Described herein is a method for determining whether a subject has facioscapulohumeral muscular dystrophy comprising determining whether the subject has a mutation in the DNMT3B gene. The method preferably comprises determining whether the FSHD subject has a mutation that affects the DNMT3B gene on at least one allele. In a preferred embodiment the inactivating mutation is a mutation in the promoter that reduces or inactivates transcription of the gene, a mutation in the protein coding sequence that reduces the activity or inactivates the protein, a mutation that reduces the stability of the mRNA or a combination thereof. In a preferred embodiment the mutation is a mutation that causes a premature stop of protein translation of a DNMT3B mRNA.

In the present invention it was found that ectopic expression of SMCHD1 did reverse an FSHD phenotype, both in FSHD1 and in FSHD2 patients. The present invention provides a composition for use in treating facioscapulohumeral muscular dystrophy (FSHD) in a subject, which composition comprises a therapeutically effective amount of a substance which increases the level and/or activity of a protein encoded by a structural maintenance of chromosomes flexible hinge domain containing 1 (*SMCHD1*) gene in cells of the subject, wherein the substance comprises a nucleic acid segment encoding a polypeptide that has an amino acid sequence identity of at least 80%, preferably at least 90%, more preferably at least 95%, most preferably 100% with the SMCHD1 protein of SEQ ID:NO 1. The invention also provides a composition for use in treating FSHD in a subject, which composition comprises a therapeutically effective amount of a substance which increases the level and/or activity of a protein encoded by a SMCHD1 gene in cells of the subject, wherein the substance mediates post-translational modification of SMCHD1 by one or more of sumoylation, ubiquitination and phosphorylation of the SMCHD1 protein;
wherein the composition comprises:
- N106 (N-(4-methoxybenzo[d]thiazol-2-yl)-5-(4-methoxyphenyl)-1.3,4-oxadiazol-2-amine),
- PR-619,
- SENP2 shRNA TRCN0000 004 577; SENP2 shRNA TRCN0000 004 579 or a combination thereof, or
- ubiquitin-like modifier activating enzyme 2 (UBA2) or an amino acid sequence that has an amino acid sequence identity of at least 80%, preferably at least 90%, more preferably at least 95%, most preferably 100%) with the UBA2 protein, or a nucleic acid segment encoding said UBA2, which increases a small ubiquitin-like modifier (SUMO)-activating enzyme level.

The composition for use according to the invention is suitable for treating subjects with an inactivating mutation in SMCHD1, i.e. FSHD type 2. The composition is also suited for the treatment of subjects with a D4Z4 repeat array contracted to a size of 1-10 units (FSHD type 1) having wild-type, or naturally functioning, SMCHD1. The composition of the invention is further suited for the treatment of subject that have a dominant negative mutation in SMCHD1. Some dominant negative mutations may have benefit from the additional expression of wild-type or active SMCHD1. In particular for the subjects with FSHD type 1 expressing wild-type, or naturally functioning, SMCHD1 it is interesting that an increase in level and/or activity of SMCHD1 protein still results in enhanced repression of DUX4 in muscular cells of the subject. Subjects that have a contracted D4Z4 repeat array and a mutation in SMCHD1 exhibit a more severe FSHD1 phenotype and are classified as a special case of FSHD1. Where in the invention a preference is mentioned for treatment of a subject with FSHD1, it is preferred that the subject does not have the above mentioned special case of FSHD1. In another preferred embodiment it is preferred that the subject does have the above mentioned special case of FSHD1. The composition for use in the present invention also works in FSHD2 caused by DNMT3B mutations and in FSHD1 DNMT3B modifier mutations.

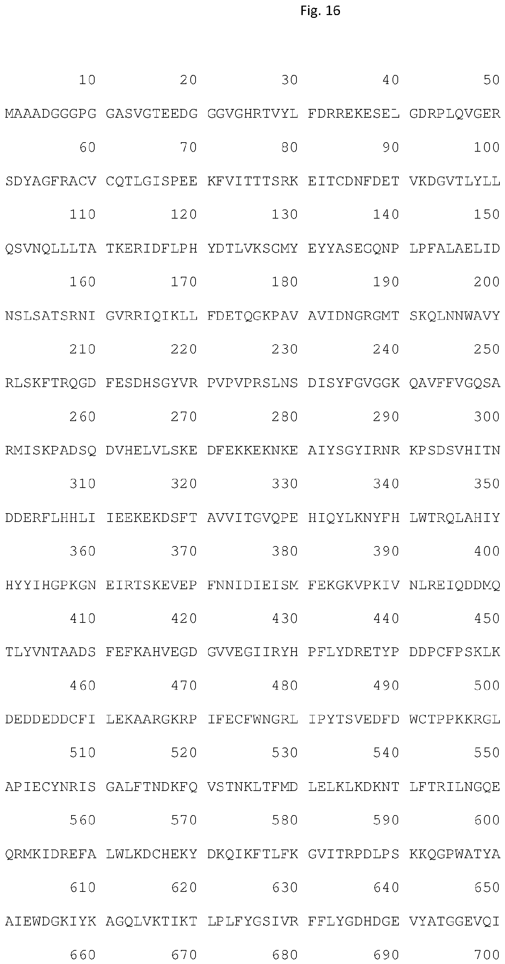

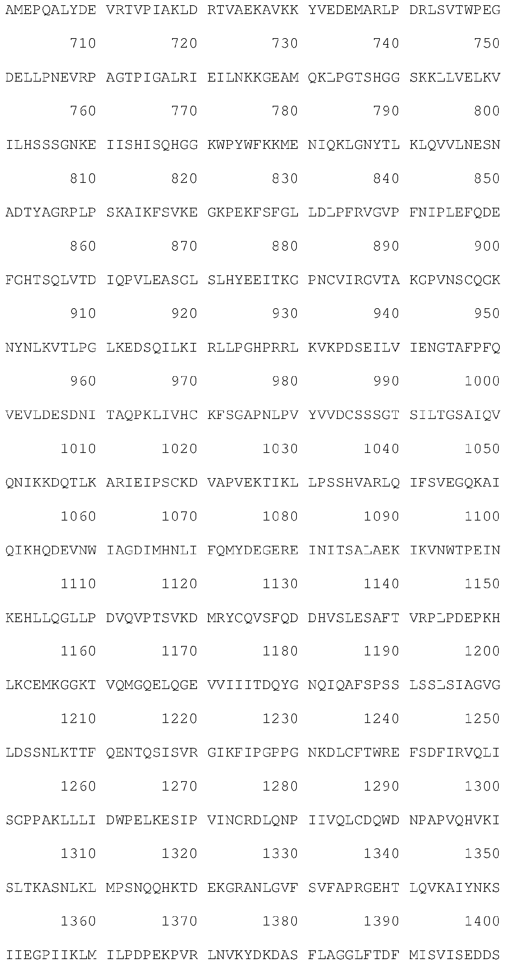

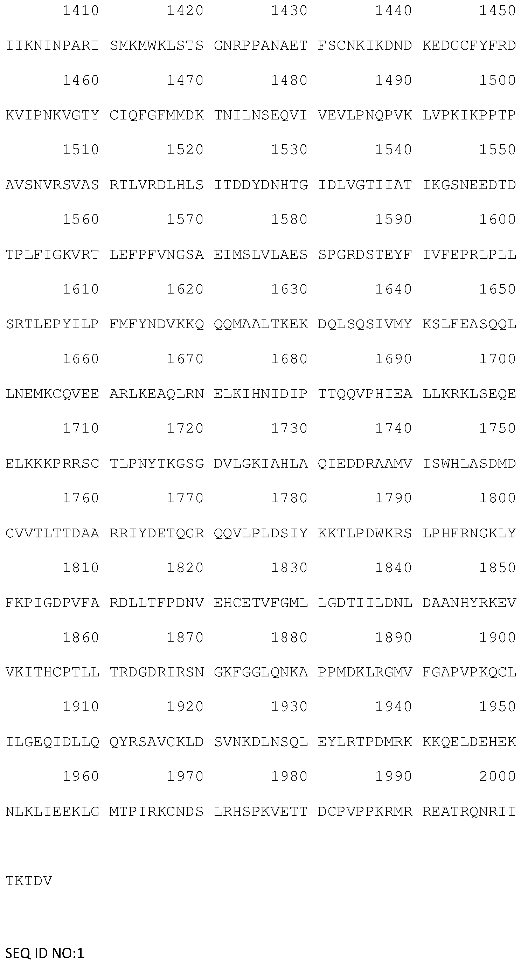

In a particular embodiment of the composition for use according to the invention, the substance increases a SMCHD1 protein level in cells of the subject. In one embodiment the substance may comprise a therapeutically effective amount of a polypeptide having an amino acid sequence of SEQ ID NO: 1 (figure 16) or an amino acid sequence that has an amino acid sequence identity of at least 80%, preferably at least 90%, more preferably at least 95%, most preferably 100% with the SMCHD1 protein of SEQ ID NO: 1. Preferably the polypeptide binds to chromosome 4q35 D4Z4 repeat array in FSHD cells of the subject. Preferably the polypeptide represses expression of DUX4.

In an alternative embodiment of the composition for use according to the invention the substance provides a nucleic acid segment encoding a polypeptide that has an amino acid sequence identity of at least 80%, preferably at least 90%, more preferably at least 95%, most preferably 100% with the SMCHD1 protein of SEQ ID NO:1, and particularly having at most a two amino acids difference with the sequence of SEQ ID NO:1. The ectopic expression of such SMCHD1 gene or nucleic acid supplements the naturally occurring expression of SMCHD1, thereby increasing a total amount or level of the SMCHD1 protein or functionally equivalent polypeptide in the cell. Ectopic expression is referred to herein as the expression of a coding sequence in an abnormal place in an organism or from an abnormal chromosomal position in the cell, and/or a sequence coding for a heterologous protein that is not normally expressed in the cell. Ectopic expression is typically achieved by artificially introducing a nucleic acid that codes for the respective expressed nucleic acid in the cell. Ectopic expression of a nucleic acid can be done by introducing a transgene with a (modified) promoter into the target cell (transient or stable transfection).

The SMCHD1 gene or the nucleic acid segment can be encoded by an expression cassette which is provided to said cell. Preferably the expression cassette encodes an RNA transcript in which the coding region for the SMCHD1 protein is contained. Particularly the expression cassette in addition to the sequence of interest, i.e. the SMCHD1 gene or the nucleic acid segment, contains additional sequences, for instance a suitable promoter and a transcription termination sequence, for controlled and/or proper expression of the sequence of interest. It is within the skill of the artisan to design suitable expression cassettes and transcripts. Suitable examples are presented in the experimental section.

A vector typically comprises a DNA molecule used as a vehicle to artificially carry foreign genetic material into another cell, where it can be replicated and/or expressed. A vector containing foreign DNA is termed recombinant DNA. The some types of vectors are plasmids, viral vectors, cosmids, and artificial chromosomes.

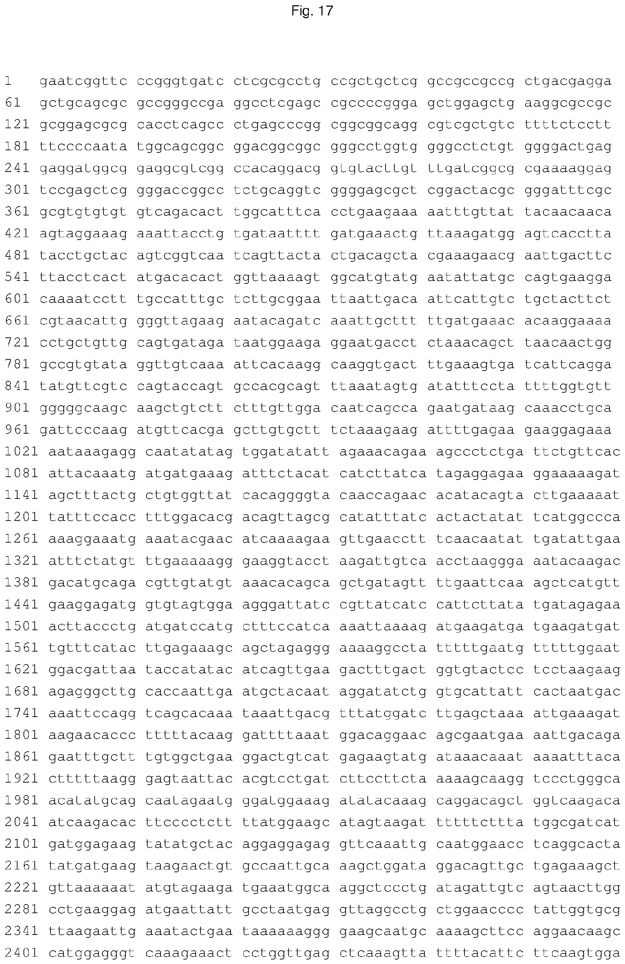

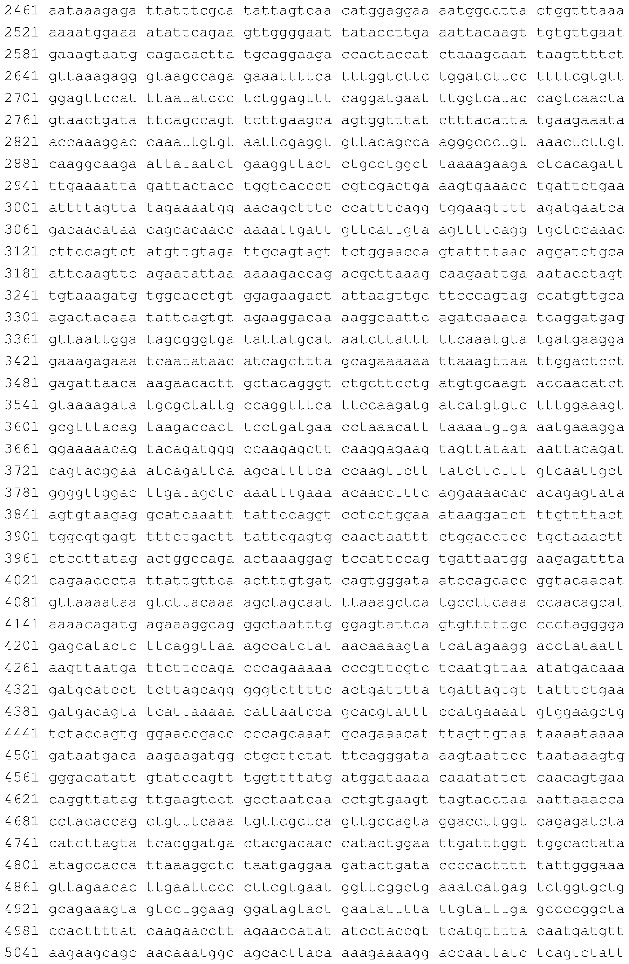

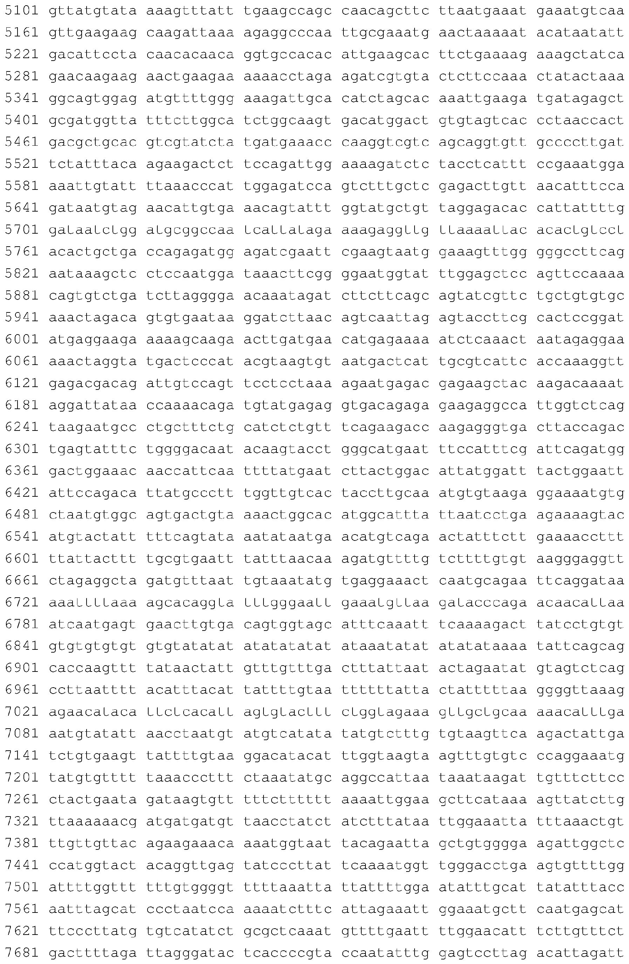

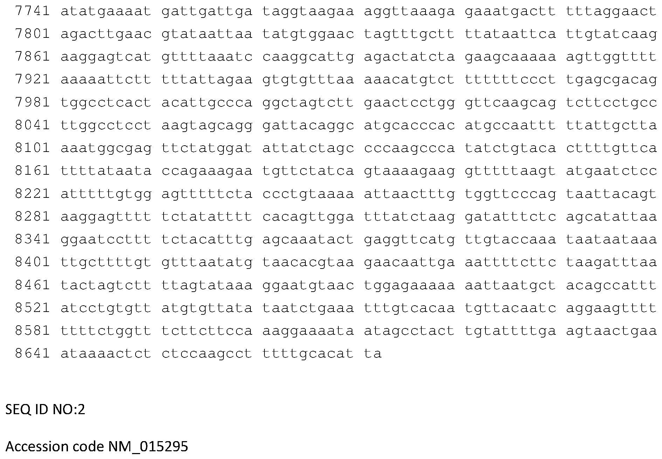

The expression cassette used is preferably a vector, preferably an expression vector. In a particular embodiment according to the present invention the substance comprises a vector which is suitable for transfecting or transducing cells of the subject and which vector carries at least one copy of a nucleic acid encoding SMCHD1. In a preferred embodiment the nucleic acid is a nucleic acid comprising the coding region of SEQ ID NO:2 (figure 17) or the nucleic acid segment. The number of copies of the gene or nucleic acid segments used may be adapted to an envisaged amount of SMCHD1 protein or functionally equivalent polypeptide to be expressed in the cells of the subject.

The expression vector is preferably suitable as a gene delivery vehicle to be introduced into the cell. Typically, the SMCHD1 gene or the nucleic acid segment is encoded by a viral or virus-based gene delivery vehicle. A preferred embodiment of the delivery vehicle is a viral vector such as an adenoviral vector and more preferably an adeno-associated virus vector. In a particular preferred embodiment the substance in the composition according to the invention comprises an adeno-associated virus vector or a lentivirus vector, more particularly a lentivirus vector carrying the SMCHD1 gene. The invention thus also provides such expression vectors and delivery vehicles carrying the SMCHD1 gene or a nucleic acid segment encoding a polypeptide that has an amino acid sequence identity of at least 80%, preferably at least 90%, more preferably at least 95%, most preferably 100% with the SMCHD1 protein.

Where herein mention is made of a SMCHD1 protein it is preferred that the protein comprises the amino acid sequence of SEQ ID NO: 1, or an amino acid sequence having an amino acid sequence identity of at least 80%, preferably at least 90%, more preferably at least 95%, most preferably 100% with the amino acid sequence of SEQ ID NO: 1. In a preferred embodiment the protein has at most a two amino acids difference with the amino acid sequence of SEQ ID NO:1. It is preferred that the protein binds to the chromosome 4q35 D4Z4 repeat array in cells, preferably in cells of a subject with FSHD. The protein preferably represses expression of DUX4. Where herein mention is made to a SMCHD1 gene, reference is made to a nucleic acid that encodes a protein as defined in this paragraph. In a preferred embodiment the gene comprises the coding region present in the sequence of SEQ ID NO: 2. The reference may be to the gene as present on the chromosome of a human cell, i.e. including the promoter and transcription signals present thereon and including the introns. The reference also includes a cDNA of SMCHD1. It typically refers to the coding region of SMCHD1 with or without one or more (artificial) introns, such as is typically used in nucleic acid molecules designed for directing heterologous expression of SMCHD1.

An expression cassette, vector or gene delivery vehicle such as a viral vector typically comprises all of the sequences that are required *in cis* to allow efficient transcription and translation of the coding region.

In a particular embodiment the substance in the composition for use according to the invention increases a SMCHD 1 protein level by enhancing expression of the SMCHD 1 gene in cells of the subject. The substance can for instance comprise a small molecule or small RNA that is capable of increasing SMCHD1 gene expression in cells of the subject, such as for instance by activating a transcription factor. The substance may in addition to such a small molecule or small RNA also comprise one or more other gene regulation peptides that promote gene transcription, such as for example a coactivator, chromatin remodeler, histone acetylase, kinase, and methylase. The enhanced expression of the SMCHD1 gene diminishes DUX4 expression levels and therefore is a suitable therapeutic method for treating the symptoms affiliated with FSHD, especially in subjects having the contracted D4Z4 repeat array to a size of 1-10 units but wild-type SMCHD1, i.e. subjects with FSHD1.

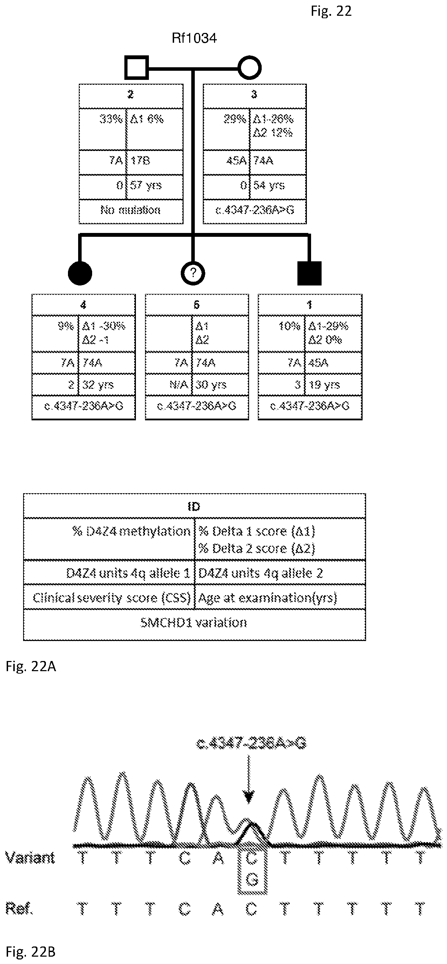

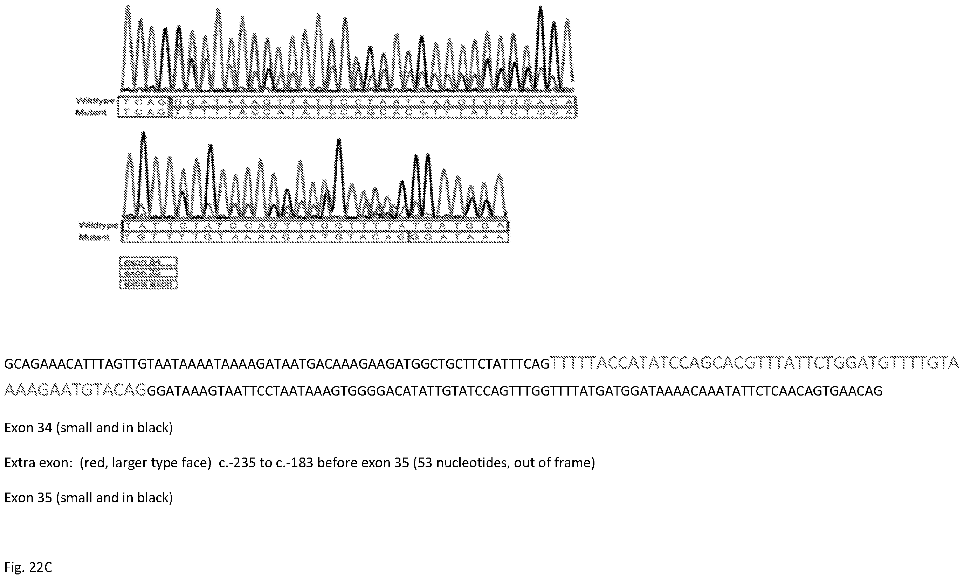

The composition for use according to the invention in an alternative embodiment comprises a substance which increases the SMCHD1 protein activity. The composition preferably comprises a substance that increases or stabilizes SMCHD1 protein binding to D4Z4 in FSHD cells of the subject. In some subjects having specific mutations in SMCHD1, either FSHD2 or the severe form of FSHD1, the activity of the SMCHD1 protein in the repression of the DUX4 gene particularly in muscle cells or progenitor muscle cells of the subject may be restored using appropriate exon-skipping. For example some subjects have FSHD because of a mutation in SMCHD1 that results in the formation of a cryptic splice site. In particular cases this leads to the addition of a 53bp exon sequence between exon 34 and exon 35 compared to wild-type SMCHD1 mRNA (Figure 22). As a result of the addition the mRNA encodes a premature stop. The mutation may be treated by exon-skipping. Skipping of the additional exon results in recovery of the in frame wild-type SMCHD1 protein. The skip may be induced by creating antisense oligonucleotides (AONs) targeted to the additional exon sequence. Preferably mutation-specific AONs are used.

The AON typically comprises a nucleic acid sequence of 15-40 nucleic acids with a sequence that comprises at least 10 and more preferably at least 15 consecutive nucleic acids complementary to the RNA sequence of the cryptic exon.

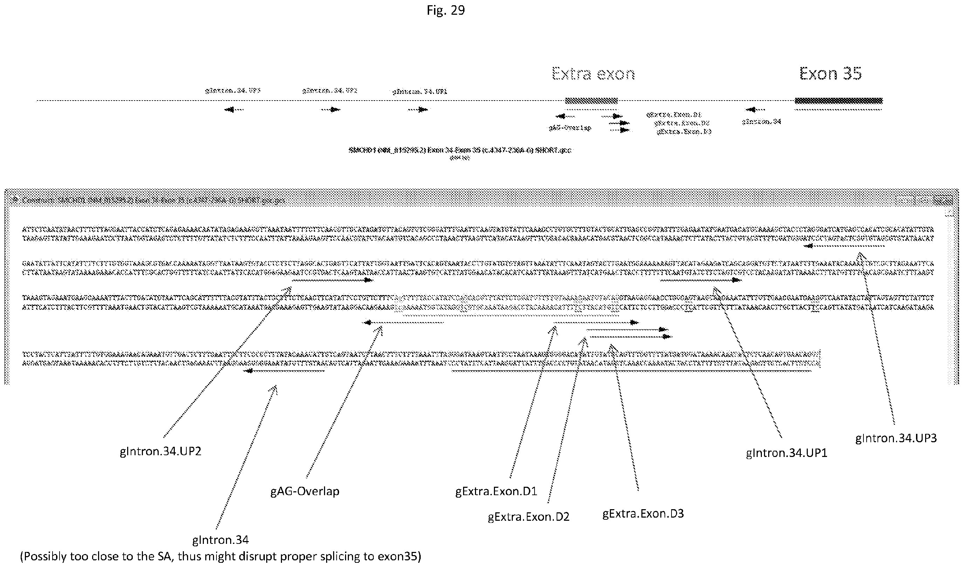

As an alternative, the CRISPR-Cas9 system may be used for exon-skipping. Exon-skipping using CRISPR-Cas9 has been reported in, e.g., Nelson et al. Science 2016 351:403; Tabebordbar et al. Science 2016 351:407, and Long et al. Science 2016 351:400 Similar methods are encompassed herein. In an exemplary embodiment, SpCas9 is cloned into an AAV vector under control of a CMV promoter. In the same or different AAV vector, an sgRNA is provided under the control of a U6 promoter. Exemplary guide RNA sequences are provided in Figure 29.

Expression of Cas9 and sgRNA results in skipping of the extra exon and restoration of the SMCHD1 reading frame.

In a further particular embodiment the composition for use according to the invention comprises a substance that affects a post-translational modification of SMCHD1 protein in FSHD cells of the subject. one or more of sumoylation, ubiquitination and phosphorylation of the SMCHD1 protein. It is found that such post-translational modification of SMCHD1 protein in FSHD cells results in lowered DUX4 expression in those cells. Particularly sumoylated SMCHD1 demonstrates an increased effect on DUX4 repression.

Thus, in a particular embodiment the substance in the composition for use according to the invention increases a SUMO-activating enzyme level in cells of the subject. In one embodiment the substance may comprise a therapeutically effective amount of ubiquitin-like modifier activating enzyme 2 (UBA2) or an amino acid sequence that has an amino acid sequence identity of at least 80%, preferably at least 90%, more preferably at least 95%, most preferably 100% with the UBA2 protein. Preferably the polypeptide stimulates sumoylation of SMCHD1. Ubiquitin-like modifier activating enzyme 2 (UBA2), also known as ubiquitin-like 1-activating enzyme E1B (UBLE1B) or SUMO-activating enzyme subunit 2 (SAE2), is an enzyme that in humans is encoded by the *UBA2* gene, external Ids HGNC:30661; Entrez Gene: 10054; Ensembl: ENSG00000126261; OMIM:613295 and UniProtKB: Q9UBT2. UBA2 forms together with SAE1 a heterodimer that functions as a SUMO-activating enzyme for the sumoylation of proteins. In an alternative embodiment of the composition for use according to the invention the substance provides a nucleic acid segment encoding a polypeptide that has an amino acid sequence identity of at least 80%, preferably at least 90%, more preferably at least 95%, most preferably 100% with the UBA2 protein. The UBA2 protein or gene is preferably a human gene. In a preferred embodiment the composition comprises a molecule that activates a SUMO-activating enzyme, such as compound N106 (N-(4-methoxybenzo[d]thiazol-2-yl)-5-(4-methoxyphenyl)-1,3,4-oxadiazol-2-amine) as described in Kho et al.Nat.Com. 2015; 12. June.

In another particular embodiment the substance in the composition for use according to the invention comprises an inhibitor of a sumo protease, and in particular comprises the compound PR-619. PR-619, a chemical compound with the molecular formula C₇H₅N₅S₂, is a non-selective, reversible inhibitor of sumo proteases. PR-619 is cell permeable and treatment of cells thus stimulates the accumulation of sumo-conjugated proteins in those cells. Use of PR-619 in a composition for use according to the invention therefore stimulates the accumulation of sumoylated SMCHD1 protein in FSHD cells and is therefore an effective means for reducing the level of expression of DUX4.

PR-619 (CAS Number 2645-32-1) is also known as 2,6-Diamino-3,5-dithiocyanopyridine, Thiocyanic acid C,C'-(2,6-diamino-3,5-pyridinediyl) ester; 2,6-Diaminopyridine-3,5-bis(thiocyanate). It can be commercially obtained from Sigma Aldrich.

Another particular embodiment of the composition for use according to the invention concerns a substance comprising an inhibitor of a sentrin-specific protease (SENP), in particular an inhibitor of SENP2 or a *SENP2* gene, a substance comprising an antisense molecule, a transcription factor, in particular a zinc finger protein, and/or a small RNA, in particular a RNAi, which reduces expression of the *SENP2* gene, the substance preferably comprises SENP2 shRNA TRCN0000 004 577; SENP2 shRNA TRCN0000 004 579 or a combination thereof (table S3). Other inhibitors are the inhibitor of SENP2, SENP2 1,2,5-Oxadiazole inhibitor as described in Kumar et al (2014) J. Chem. Inf. Model. Vol. 54, 870-880 and the inhibitor compound 69 or compound 117 as described in Kumar et al (supra).

Mammals contain seven SENPs, i.e. SENP1, SENP2, SENP3, SENP5, SENP6, SENP7, and SENP8), of which SENP1, 2, 3, 5, 6 and 7 are known SUMO proteases. SENP8 is known to function on another small ubiquitin-related protein, i.e. Nedd8. The SENPs are involved in the maturation of SUMO precursors by removing C-terminal extensions, i.e. endopeptidase cleavage, and deconjugation of the targets, i.e. isopeptidase cleavage. Thus, SENPs are an important factor for sumoylation pathways in cells. Sentrin-specific protease 2 (SENP2) is a human cysteine protease of 589 amino acids and a weight of 68 kDa encoded by the *SENP2* gene, external Ids are HGNC:23116; Entrez Gene: 59343; Ensembl: ENSG00000163904; OMIM:608261 and UniProtKB: Q9HC62. SENP2 catalyzes the proteolysis of SUMO1 and SUMO2/3 and has a broad substrate specificity. Use of a SENP2 shRNA, in a composition for use according to the invention therefore reduces isopeptidase cleavage of SUMO from substrates such as SMCHD1 in the cell, and is therefore an effective means for reducing the level of expression of DUX4 in FSHD cells. Suitable SENP inhibitors include the following compounds as described in Kumar, Zhang. Comp. and Struct. Biotech. J. 2015; 13, 204-211, compound 9 (SENP1 inhibitor), compound 11 (SENP1, SENP2, and SENP7 inhibitor), compound 13 (SENP1 and SENP2 inhibitor), and compound 15 (SENP1 inhibitor).

RNF4 is also known as E3 Ubiquitin Ligase RNF4; Small Nuclear Ring Finger Protein; E3 Ubiquitin-Protein Ligase RNF4; SNURF; EC 6.3.2.; SLX5 and Protein SNURF. External Ids are HGNC: 10067; Entrez Gene: 6047; Ensembl: ENSG00000063978; OMIM: 602850 and UniProtKB: P78317.

Although one of the substances described in the foregoing may be used in a composition for use according to the invention for treating FSHD, it is also envisaged that any combination of two or more of such substances are used in the composition for use according to the invention. Accordingly in a preferred embodiment the composition for use in treating facioscapulohumeral muscular dystrophy (FSHD), in particular FSHD1, in a subject, comprises one or more of the foregoing substances which increase a level of SMCHD1 protein in muscle cells and/or one or more of the foregoing substances that increase protein activity of SMCHD1 in such muscle cells. For instance a composition comprising both an amount of a polypeptide that has an amino acid sequence identity of at least 80%, preferably at least 90%, more preferably at least 95%, most preferably 100% with the SMCHD1 protein, wherein the polypeptide is preferably relatively highly sumoylated, as well as an effective amount of PR-619 may be used in treating facioscapulohumeral muscular dystrophy (FSHD) in a subject. However, any other suitable combination of two or more of the substances mentioned herein may be taken to prepare the composition for use according to the present invention.

Also described is a method of preparing SMCHD 1 protein or a polypeptide that has an amino acid sequence identity of at least 80%, preferably at least 90%, more preferably at least 95%, most preferably 100% with the SMCHD1 protein for use in a composition for use according to the invention, wherein a post-translational modification step is applied to the SMCHD1 protein or the polypeptide, the step comprising one or more of sumoylation, de-ubiquitination and phosphorylation of the SMCHD 1 protein.

In a preferred embodiment of the method according to the invention the FSHD is of the type facioscapulohumeral muscular dystrophy 1 (FSHD1). In another preferred embodiment of the invention the FSHD is of the type FSHD2.

In a further preferred embodiment of the method according to the invention the substance is capable of increasing SMCHD1 protein level in FSHD cells of the subject via ectopic expression of a SMCHD1 gene or a nucleic acid segment encoding a polypeptide that has an amino acid sequence identity of at least 80%, preferably at least 90%, more preferably at least 95%, with the SMCHD1 protein in cells of the subject, and particularly having at most a two amino acids difference therewith.

In a particular embodiment of the method according to the invention the substance comprises a vector which is suitable for transfecting skeletal muscle cells or progenitor muscle cells of the subject and carries at least one copy of the SMCHD1 gene or the nucleic acid segment.

In a further particular embodiment of the method according to the invention an adeno-associated virus vector or a lentivirus vector carrying at least one copy of the SMCHD1 gene or the nucleic acid segment is used for transfecting skeletal muscle cells or progenitor muscle cells of the subject, more particularly a lentivirus vector carrying the SMCHD1 gene is used.

In a further particular embodiment of the method according to the invention the substance increases SMCHD1 protein level in skeletal muscle cells or progenitor muscle cells of the subject by enhancing expression of the wild-type SMCHD1 gene. A further preferred embodiment of the method according to the invention has as a feature that the substance comprises a small molecule or small RNA that is capable of increasing SMCHD1 gene expression in muscle cells or progenitor muscle cells of the subject.

In another particular embodiment of the method according to the invention the substance is SMCHD1 protein or a polypeptide that has an amino acid sequence identity of at least 80%, preferably at least 90%, more preferably at least 95%, with the SMCHD1 protein, which is capable of binding to chromosome 4q35 D4Z4 repeat array in FSHD cells of the subject, which protein or polypeptide is administered to the subject using a pharmaceutically acceptable carrier.

In a further particular embodiment of the method according to the invention the substance is administered to the subject in an amount that provides for an amount of active SMCHD1 protein in muscle cells or progenitor muscle cells of the subject at least equal to the regular average amount of SMCHD1 protein as found in cells of subjects not suffering from FSHD, and preferably larger than the regular average amount.

In an alternative embodiment of the method according to the invention the substance increases the SMCHD1 protein activity by stimulating activity of SMCHD1 protein in the repression of the DUX4 gene particularly in muscle cells or progenitor muscle cells of the subject. In a particular embodiment hereof the method according to the invention has the feature that the substance increases or stabilizes SMCHD1 protein binding to D4Z4 particularly in muscle cells or progenitor muscle cells of the subject. In some subjects having specific mutations in SMCHD1, either FSHD2 or the severe form of FSHD1, the activity of the SMCHD1 protein in the repression of the DUX4 gene particularly in muscle cells or progenitor muscle cells of the subject may be restored using exon-skipping.

In a further particular embodiment of the method according to the invention the administered substance affects post-translational modification of SMCHD1 protein particularly in muscle cells or progenitor muscle cells of the subject. Another particular embodiment of the method according to the invention has the feature that the substance stimulates one or more of sumoylation, de-ubiquitination and phosphorylation of the SMCHD1 protein.

In a further particular embodiment of the method according to the invention the administered substance comprises an inhibitor of sumo proteases, and in particular comprises PR-619.

Although one of the substances described in the foregoing may be used in treating FSHD according to the invention, it is also envisaged that any combination of two or more of such substances are used in the method according to the invention. Accordingly in a preferred embodiment the method of treating facioscapulohumeral muscular dystrophy (FSHD), particularly FSHD1, in a subject has the feature that a therapeutically effective amount of at least one of the in the foregoing described substances that increase a level of SMCHD1 in muscle cells or progenitor muscle cells of the subject is administered to the subject and/or at least one of the in the foregoing described substances that increases an activity of SMCHD1 in muscle cells or progenitor muscle cells of the subject is administered to the subject.

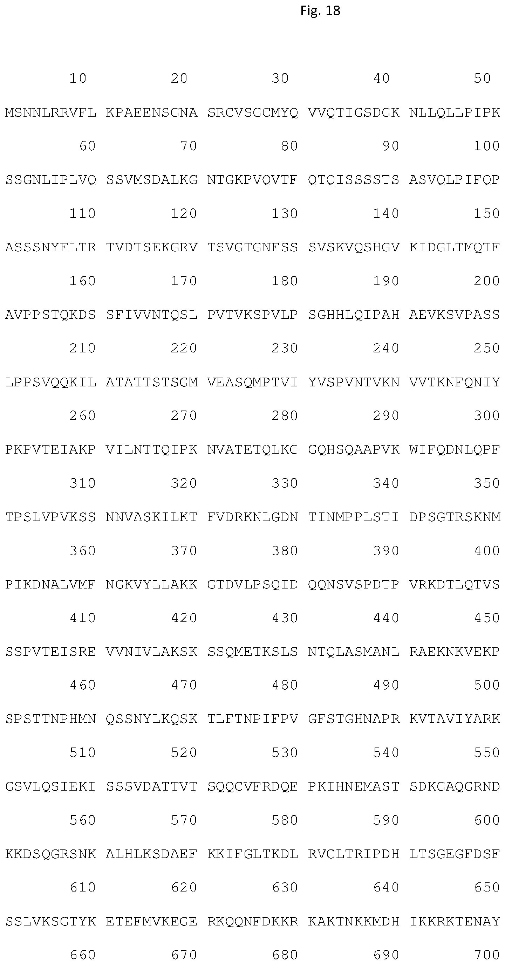

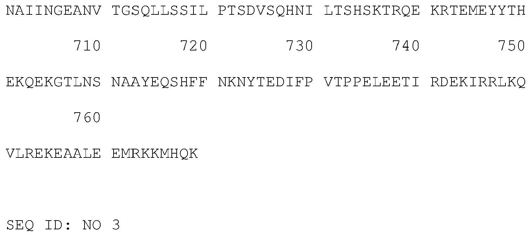

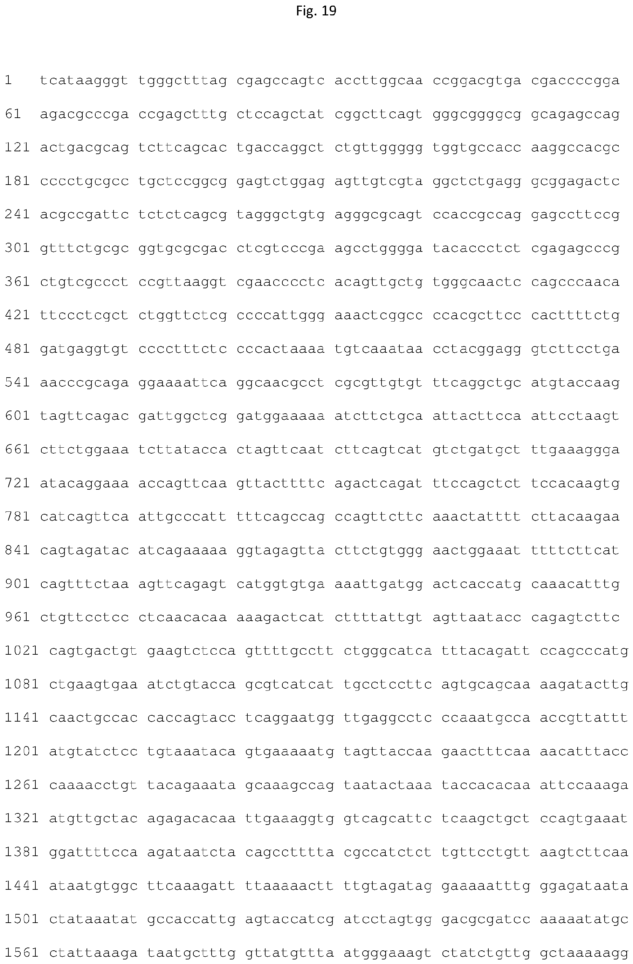

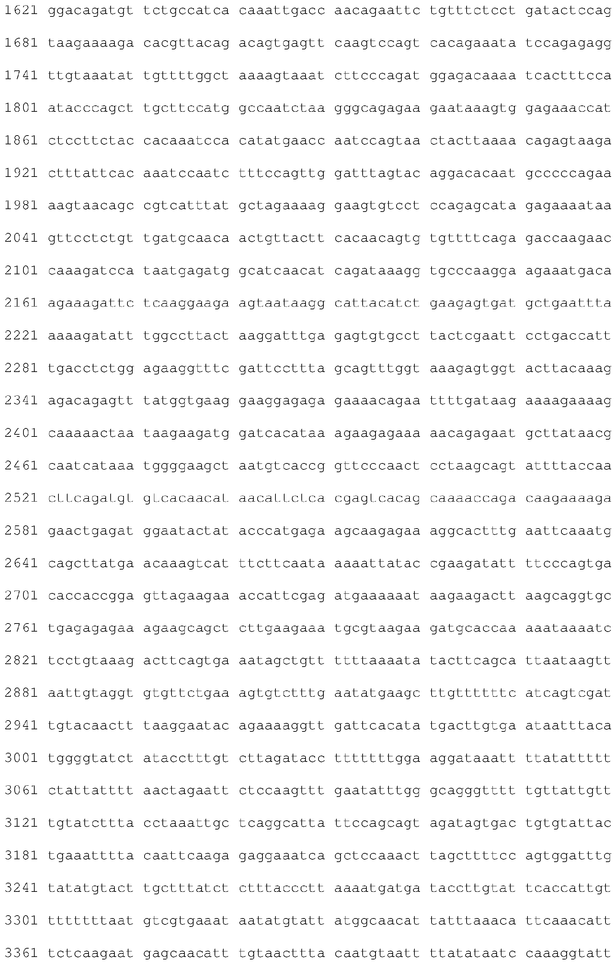

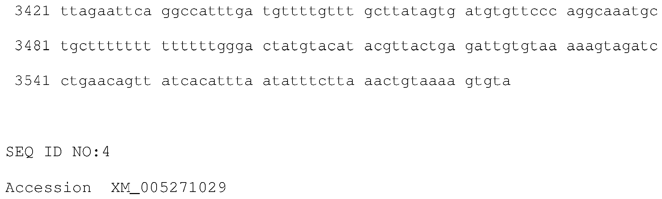

In another aspect the present invention may provide a composition for treating FSHD in a subject which involves, besides a substance that increases the level and/or activity of a protein encoded by a structural maintenance of chromosomes flexible hinge domain containing 1 (*SMCHD1*) gene or a functionally equivalent polypeptide thereof, the use respectively administration of any other substance that reduces DUX4 expression in muscle cells or muscle progenitor cells in the subject. Also disclosed is a composition for use in the treatment of an individual having FSHD wherein said composition comprises a substance that reduces the level of LRIF1 in a muscle cell of the subject. Also disclosed is a method for the treatment of a subject having FSHD comprising administering to the subject in need thereof a substance that reduces the level of LRIF1 in a muscle cell of the subject. The substance that reduces the level of LRIF1 in a cell preferably comprises an antisense oligonucleotide (AON) that is complementary to and capable of hybridizing with LRIF1 RNA. The antisense oligonucleotide may be a small RNA, in particular a RNAi or an exon skip AON. In another embodiment the substance that reduces the level of LRIF1 in a cell comprises a gene delivery vehicle with a nucleic acid molecule that codes for the antisense oligonucleotide. In this preferred embodiment the nucleic acid molecule preferably encodes an shRNA specific for LRIF1 mRNA. In an embodiment a substance or composition comprises a gene delivery vehicle encoding an shRNA against Chromosome 1 open reading frame 103 (C1orf103) gene of SEQ ID NO:4 (figure 19) and capable of expressing said shRNA in muscle cells or muscle progenitor cells in a subject. In a preferred embodiment the shRNA is an shRNA of table s3. For instance in an embodiment of this aspect a composition comprising as a substance a vector carrying a copy of an shRNA against Chromosome 1 open reading frame 103 (C1orf103) gene of SEQ ID NO:4 (figure 19) and capable of expressing said shRNA in muscle cells or muscle progenitor cells in a subject. The C1orf103 gene in humans codes for the protein ligand-dependent nuclear receptor-interacting factor 1 (LRIF1) SEQ ID NO:3 (figure 18). IDs in databases of the human protein and genes are HGNC: 30299;; Entrez Gene: 55791; Ensembl: ENSG00000121931; OMIM: 615354; and UniProtKB: Q5T3J3. A nucleotide sequence of a cDNA encoding LRIF1 is presented in SEQ ID NO:4 (figure 19). A LRIF1 protein is believed to be an interaction partner of SMCHD1. It was investigated whether LRIF1 levels influence DUX4 expression. Using a lentivirus carrying a shRNA against LRIF1 it was shown that in cells transduced with the lentivirus LRIF1 was depleted. In germline cells the depletion of LRIF1 was associated with an increased transcription of SMCHD1 and a decreased transcription of DUX4 in those cells. In myoblast cells with depleted LRIF no statistically relevant increase in SMCHD1 transcription was observed, however it was observed that DUX4 transcription was decreased. These findings indicate that silencing of LRIF1 in muscle cells or progenitor muscle cells of subjects with FSHD is a suitable way of reducing DUX4 expression, and therefore to treat FSHD symptoms in subjects with either FSHD1 or FSHD2.

Instead of being used in the composition in addition to a substance that increases the level and/or activity of a protein encoded by a structural maintenance of chromosomes flexible hinge domain containing 1 (*SMCHD1*) gene or a functionally equivalent polypeptide thereof, the composition comprising a vector carrying a copy of an shRNA against C1orf103 or a functionally equivalent nucleic acid may also be used in another, separate, composition, which other composition may be used in a treatment of a subject with FSHD1 or FSHD2 and may be administered to the subject with or without the composition comprising a substance that increases the level and/or activity of a protein encoded by a structural maintenance of chromosomes flexible hinge domain containing 1 (*SMCHD1*) gene or a functionally equivalent polypeptide thereof.

### DESCRIPTION OF THE DRAWINGS

These and other aspects of the present invention are further elucidated by the appended drawings, which form part of the present application. The drawings are not in any way meant to reflect a limitation of the scope of the invention, unless this is clearly and explicitly indicated.
Figure 1 illustrates that DUX4 activation during myogenic differentiation coincides with SMCHD1 reduction.
   Fig.1A) Immunofluorescence microscopy analysis confirmed the typical DUX4 protein expression pattern in myosin positive, multinucleated myotubes derived from FSHD1 and FSHD2 individuals. Images were taken using a 200x magnification, scale bars are displayed.

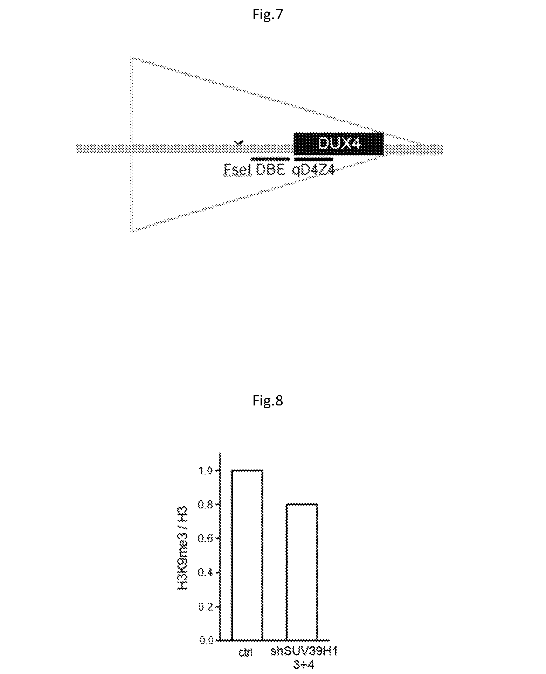

   Fig.1B) Quantitative mRNA analysis in control (C), FSHD1 (F1) and FSHD2 (F2) primary myoblasts (B) and myotubes (T) showing increased levels of DUX4 expression upon differentiation. *GAPDH* and *GUSB* were used as reference genes, *n* indicates number of samples, error bars display SEM and significance was calculated using a two tailed student's t-test.
   Fig. 1C) Quantitative mRNA analysis showed robust ZSCAN4 activation upon myogenic differentiation in FSHD myotubes. ZSCAN4 is a direct target of the DUX4 transcription factor. *GAPDH* and *GUSB* were used as reference genes, *n* indicates number of samples, error bars display SEM and significance was calculated using a two tailed student's t-test.
   Fig. 1D) Western blot analysis showing reduced levels of SMCHD1 upon muscle cell differentiation in a primary muscle cell culture derived from a control individual. Myoblasts (B) were differentiated into myotubes (T) for 48 h. H3 serves as a control for equal protein loading, -ACTIN serves as a control for the induction of myogenic differentiation.
   Fig. 1E) Western blot analysis of a control derived primary fibroblast undergoing forced myogenesis by ectopic MyoD expression for 48, 72 or 94 h showed a decrease in SMCHD1 protein expression. TUBULIN serves as a loading control.
   Fig. 1F) RT-PCR analysis of DUX4 expression upon ectopic MyoD expression in control, FSHD1 or FSHD2 fibroblast revealed DUX4 activation in patient cells exclusively. Ectopic GFP expression was used as a control and GUSB serves as an internal PCR control.
   Fig.1G, 1H) Normalized ChIP qPCR analysis of SMCHD1 binding at D4Z4 in isogenic fibroblasts (F), myoblasts (B) and myotubes (T) derived from the same control individual (panel G) and two independent control derived myoblast - myotube pairs showed the highest SMCHD1 abundance in fibroblasts with a further decrease during myogenesis.
Figure 2 illustrates that SMCHD1, but not SUV39H1 and Cohesin, regulates DUX4 expression
   Fig. 2A,2B, 2D, 2E) Western blot confirmation of respectively SMCHD1, SUV39H1, RAD21, and SMC3 knockdown in control myotubes expressing the indicated lentiviral transduced shRNAs. Tubulin was used as a loading control. Representative blots of at least duplicate experiments are shown.
   Fig. 2C, 2F) Standard gel electrophoresis analysis of DUX4 expression by RT-PCR upon lentiviral knockdown of SMCHD1 and SUV39H1, respectively RAD21 and SMC3 in control myotubes. Only depletion of SMCHD1 resulted in reproducible activation of DUX4 transcription. Representative gel photos are shown of at least duplicate experiments. GUSB serves as control.
   Fig. 2G) Western blot analysis confirms a two- threefold increase of SMCHD1 expression upon its lentiviral transduction in FSHD myotubes. GFP transduced myotubes served as a negative control. Numbers above the blots indicate normalized relative expression levels of SMCHD 1 using TUBULIN as a loading control.
   Fig. 2H) Normalized mRNA expression analysis of *DUX4* showed 70-90% reduction upon ectopic expression of SMCHD1 in FSHD1 and FSHD2 myotubes.
   Fig .2I) normalized mRNA expression analysis of the DUX4 target genes *RFPL2, ZSCAN4,* and *TRIM43* showed a 70-80% reduction upon ectopic expression of SMCHD1 in FSHD1 and FSHD2 myotubes, concordant with decreased DUX4 protein expression. For panel H, I: statistical analysis was omitted as expression levels of GFP expressing myotubes were set to 1 in all individual experiments, error bars display the SEM of at least duplicate experiments.

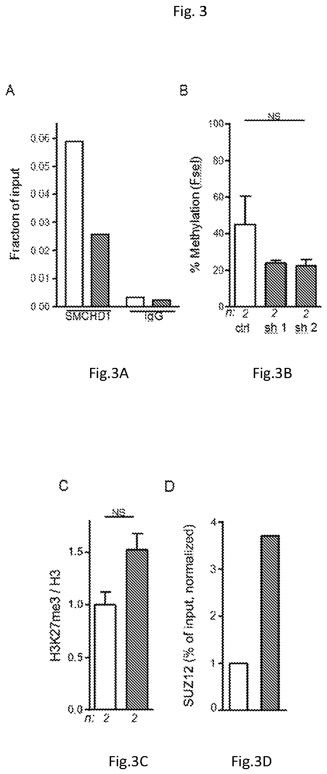

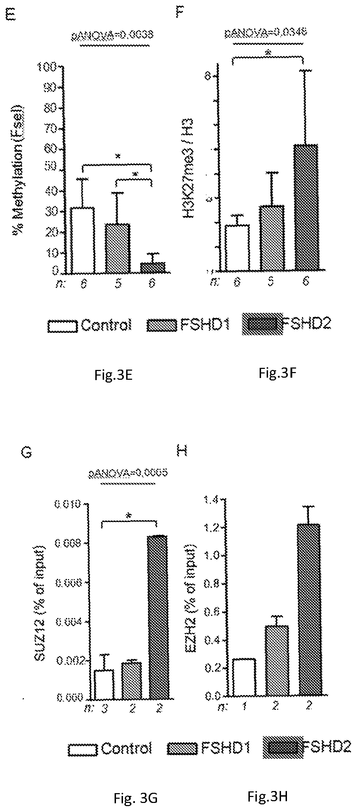

Figure 3 shows that SMCHD1 depletion at D4Z4 leads to reduced CpG methylation and increased H3k27me3 and PRC2 binding.
   Fig. 3A) ChIP-qPCR analysis showed a more than twofold reduction of SMCHD1 at qD4Z4 upon its lentiviral knockdown in control myotubes. IgG enrichment of qD4Z4 is displayed as a measure for background.
   Fig. 3B) Southern blot based quantification of FseI methylation (D4Z4) showed a twofold, though non-significant reduction of CpG methylation at D4Z4 upon depletion of SMCHD1. Error bars indicate the SEM of two independent experiments, significance was tested using a one way-ANOVA.
   Fig. 3C) ChIP-qPCR analysis of H3K27me3 at qD4Z4 upon SMCHD1 depletion showed a trend towards an increase. Enrichment values were normalized to H3 enrichment values, n indicates number of replicate experiments, error bars display SEM and significance was tested using a two tailed student's t-test.
   Fig. 3D) Normalized ChIP-qPCR analysis of SUZ12 upon SMCHD1 depletion showed a more than threefold increase at qD4Z4.
   Fig. 3E) Southern blot based quantification of DNA methylation at FseI (D4Z4) in DNA derived from myotubes. Methylation levels are significantly lower in FSHD2, with similar trend in FSHD1. N indicates sample size, error bars indicate SD and significance was tested using a one way-ANOVA followed by post hoc pairwise comparison using bonferroni correction.
   Fig. 3F) ChIP-qPCR analysis of H3K27me3 at qD4Z4 showed a significant increase in FSHD2 myotubes compared to controls. Enrichment values were normalized to H3 enrichment values, n indicates sample size, error bars display SD and significance was tested using a one way-ANOVA followed by post hoc pairwise comparison using bonferroni correction.
   Fig. 3G) ChIP-qPCR analysis of PRC2 subunit SUZ12 showed a significant increase in FSHD2 myotubes at qD4Z4. n indicates sample size, error bars display SD and significance was tested using a one way-ANOVA followed by post hoc pairwise comparison using bonferroni correction.
   Fig. 3H) ChIP-qPCR analysis of PRC2 subunit EZH2 at qD4Z4 showed a trend towards an increase in FSHD myotubes compared to controls. N indicates sample size, error bars display SD, significance was tested using a one way-ANOVA.

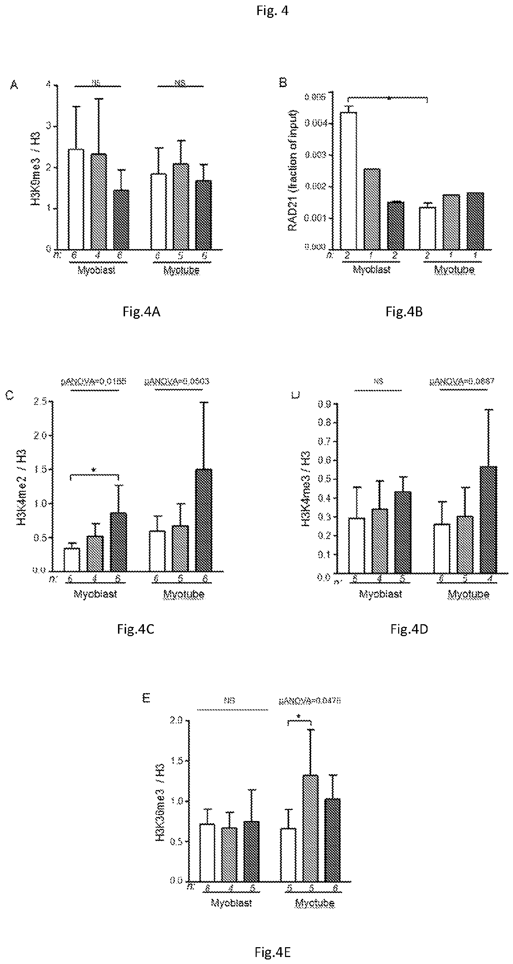

Figure 4 shows that DUX4 activation in FSHD myotubes is reflected in markers for active chromatin at D4Z4. In all panels, controls are represented in white bars, FSHD1 in grey bars and FSHD2 in black bars.
   Fig. 4A) ChIP-qPCR quantification of H3K9me3 at qD4Z4 in control and FSHD derived myoblasts and myotubes revealed no significant difference between control, FSHD1 and FSHD2.
   Fig. 4B) ChIP-qPCRanalysis of the Cohesin subunit RAD21 at qD4Z4 showed a decreased abundance in FSHD myoblasts, but not in myotubes. *n* indicates sample size, error bars display SD, asterisk indicates significance based on a two tailed student's t-test (control myoblasts vs control myotube).
   Fig. 4C) ChIP-qPCR analysis showed a significant increase in H3K4me2 at qD4Z4 in FSHD2 myoblasts compared to controls, whereas FSHD1 myoblasts showed intermediate levels. A similar trend was observed in myotubes.
   Fig. 4D) ChIP-qPCR analysis of H3K4me3 at qD4Z4 showed no difference between control and FSHD myoblasts. A trend towards an increase was observed in FSHD2 myotubes compared to controls.
   Fig. 4E) ChIP-qPCR analysis of H3K36me3 levels at qD4Z4 are not different in myoblasts, but significantly increase in FSHD1 myotubes compared to controls. H3K36me3 at qD4Z4 in FSHD2 myotubes showed a similar trend. For panels A, C-E: enrichment values were normalized to H3 enrichment values, *n* indicates sample size, error bars display SD and significance was tested using a one way-ANOVA (p values indicated) followed by post hoc pairwise comparison using bonferroni correction.

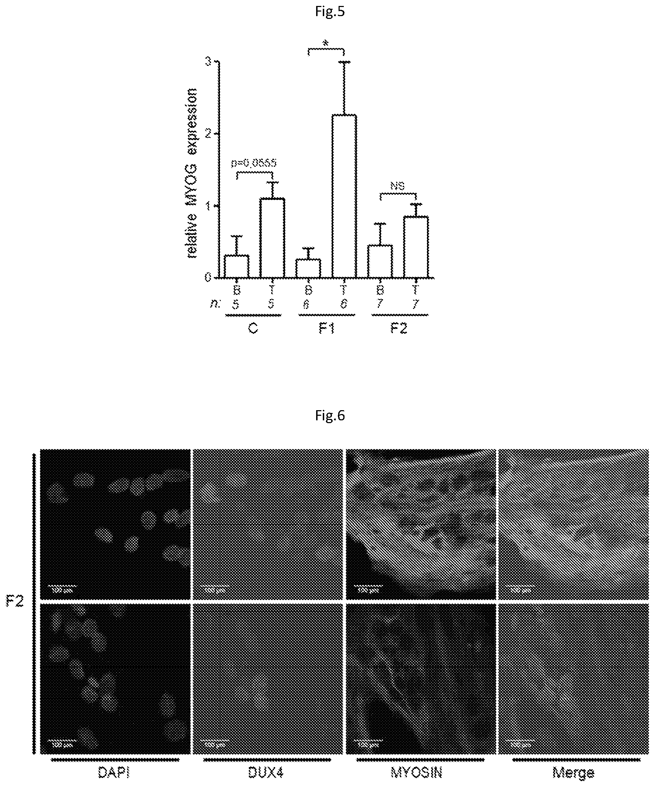

Figure 5 illustrates MYOG induction upon myogenic differentiation. mRNA expression analysis in control (C), FSHD1 (F1) and FSHD2 (F2) primary myoblasts (B) and myotubes (T) showed increased levels of MYOG expression upon differentiation in FSHD1 samples, with a similar trend in controls and FSHD2 samples. *GAPDH* and *GUSB* were used as reference genes, *n* indicates sample number, error bars display SD and significance was calculated using a two-tailed student's t-test.
Figure 6 shows an immunofluorescence analysis of sporadic DUX4 expression in FSHD2 myotubes.
   Immunofluorescence microscopy analysis confirmed the typical DUX4 protein expression pattern in myosin positive, multinucleated myotubes derived from FSHD1 and FSHD2 patients. Images were taken using a 200x magnification, scale bars are displayed. Figure 7 shows a graphical overview of the D4Z4 repeat unit and the DUX4 open reading frame.
   qD4Z4 indicates the site at the 5' end of *DUX4* used for ChIP-qPCR analyses throughout this study, the FseI site was used for methylation analyses. The location of DBE is indicated for comparison.
Figure 8 illustrates H3K9me3 levels at qD4Z4 upon depletion of SUV39H1 in control myotubes.
   ChIP-qPCR quantification of H3K9me3 at qD4Z4 showed a moderate decrease upon knockdown of SUV39H1 combining two shRNA constructs. Enrichment values were normalized to H3 enrichment values.

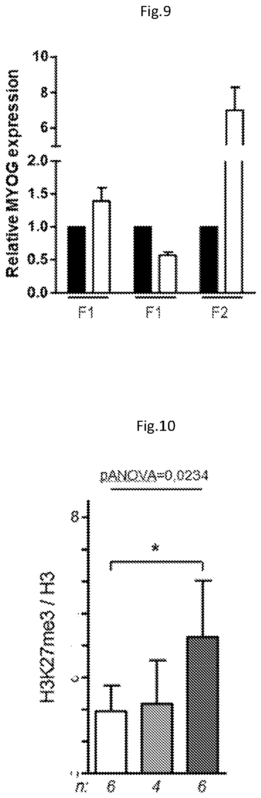

Figure 9 shows MYOG expression levels upon SMCHD 1 overexpression.
   Normalized mRNA expression analysis of *MYOG,* a marker for myogenic differentiation, showed variable levels in response to ectopic SMCHD1 expression in FSHD1 myotubes and an increase in FSHD2 myotubes. Black bars represent myoblasts, open bars represent myotubes.
Figure 10 illustrates higher levels of H3K27me3 in FSHD2 derived myoblasts.
   ChIP-qPCR analysis of H3K27me3 at qD4Z4 showed a significant increase in FSHD2 myoblasts (black bars) compared to controls (white bars). Enrichment values were normalized to H3 enrichment values, *n* indicates sample size, error bars display SD and significance was tested using a one way-ANOVA followed by post hoc pairwise comparison using bonferroni correction. FSHD1 is in grey bars.

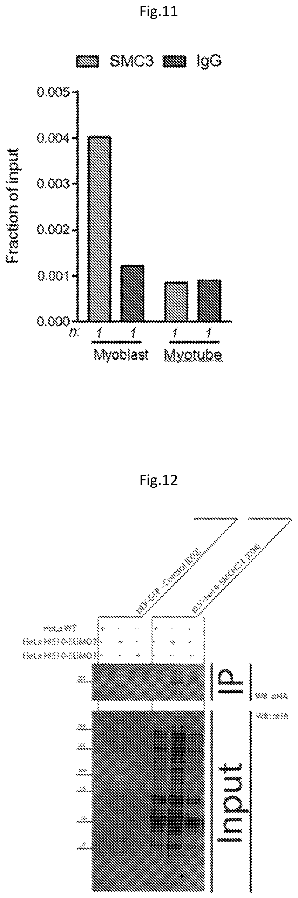

Figure 11 illustrates reduced SMC3 levels upon myogenesis in a control muscle cell culture.
   ChIP-qPCR analysis of SMC3 at qD4Z4 in a control myoblast and myotube cell culture showed reduced Cohesin levels at qD4Z4 upon myogenesis. Error bars display SD, IgG enrichment of qD4Z4 is displayed as a measure for background.
Figure 12 Western blot analysis showing immunoprecipitated SMCHD1 protein in HIS₁₀-SUMO1 and HIS₁₀-SUMO2 HeLa lysate.
   Stable HeLa cell lines containing HIS₁₀-SUMO1 or HIS₁₀-SUMO2 were transfected with HA tagged SMCHD1 expression construct. After lysis of the cells immunoprecipitation was performed using Ni-NTA agarose beads, which specifically and robustly bind HIS tagged proteins and the immunoprecipitated SMCHD1 protein was detected by Western blot analysis using HA antibodies. A protein of 200 kDa in size was detected, corresponding to the expected size of the SMCHD1 protein in the IP of HIS₁₀-SUMO1 and HIS₁₀-SUMO2 HeLa lysate.

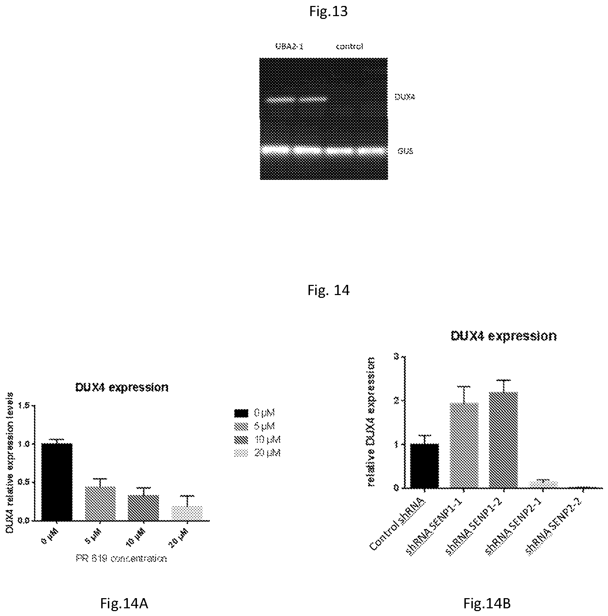

Figure 13 DUX4 quantification by RT-PCR upon downregulation of UBA2 by two distinct shRNAs.
   Depletion of UBA2 was performed by lentiviral shRNA transductions, which are efficient in primary myoblasts cultures. Cells harbouring the shRNA constructs were enriched by a selection marker present in the shRNA contructs and when cultures reached 70-80% confluency control myoblasts were differentiated into myotubes. Downregulation of UBA2 by two distinct shRNAs was confirmed on RNA and protein level and DUX4 was quantified by RT-qPCR. Shown in the figure is the upregulation of DUX4 upon UBA2 knockdown. GUS was used as control.
Figure 14 shows DUX4 transcript levels in FSHD primary muscle cell cultures. Figure 14A shows results of cell cultures treated with different doses of a non-selective inhibitor of deubiquitinases and ubiquitin-like isopeptidases PR-619. DUX4 transcript levels decreased in cells treated with PR-619 in a dose dependent manner. Figure 14B shows results of DUX4 expression in FSHD primary muscle cells with inhibition of either SENP1 or SENP2 using the SENP1 or SENP2 shRNAs indicated in Table S3.

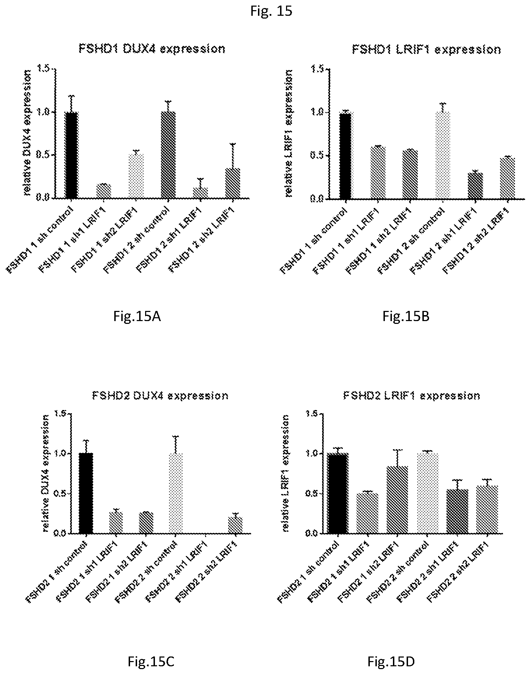

Figure 15 shows LRIF1/HBiX1/C1orf103 and DUX4 expression by qRT-PCR in two independent FSHD1 (1_1 and 1_2) and two independent FSHD2 (2_1 and 2_2) myotube cultures treated with either scrambled shRNAs (control) or one of two different anti LRIF1 shRNAs (sh1 and sh2).
   Fig. 15A) DUX4 expression in FSHD1 myotube culture;
   Fig. 15B) LRIF1 expression in FSHD1 myotube culture;
   Fig. 15C) DUX4 expression in FSHD2 myotube culture; and
   Fig. 15D) LRIF1 expression in FSHD2 myotube culture.
Figure 16 depicts the amino acid sequence (SEQ ID NO:1) corresponding to a full length human SMCHD1 protein, natural variants of this protein exist.
Figure 17 depicts the nucleic acid sequence (SEQ ID NO:2) corresponding to a full length SMCHD1 cDNA of a human SMCHD1.
Figure 18 depicts the amino acid sequence (SEQ ID NO:3) corresponding to a full length human LRIF1, natural variants of this protein exist.
Figure 19 depicts the nucleic acid sequence (SEQ ID NO:4) corresponding to a full length cDNA of a human LRIF1.

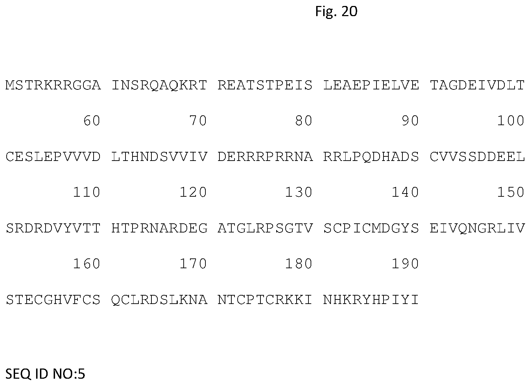

Figure 20 depicts the amino acid sequence (SEQ ID NO:5) corresponding to a full length human RNF4, natural variants of this protein exist.

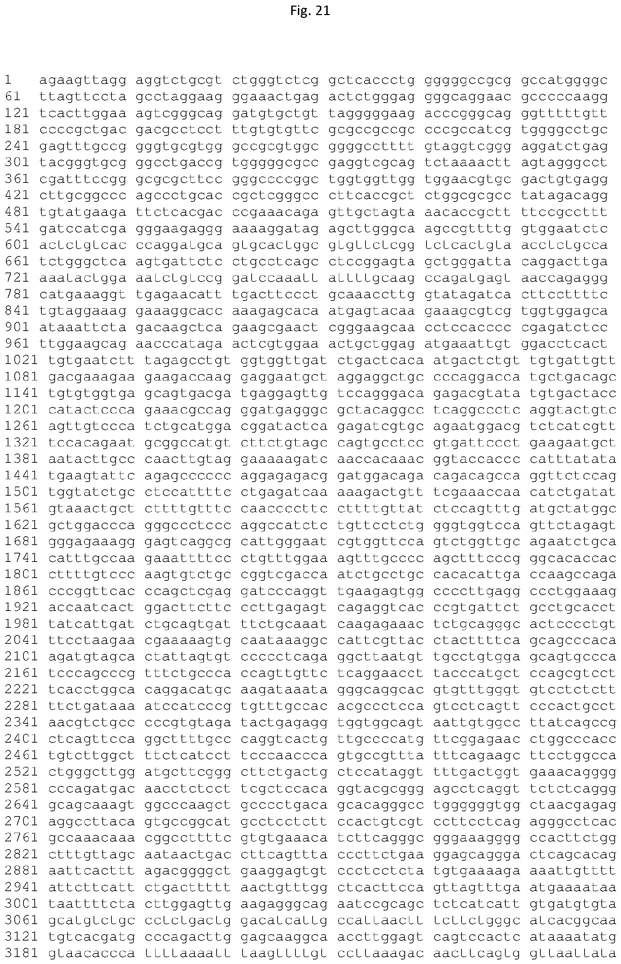

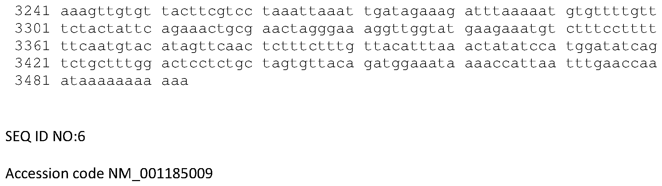

Figure 21 depicts the nucleic acid sequence (SEQ ID NO:6) corresponding to a full length cDNA of a human RNF4.
Figure 22 FSHD2 family with an intronic mutation leading to the inclusion of a cryptic exon. It shows the pedigree (A), the mutation (B) and the sequence of the new exon (C).

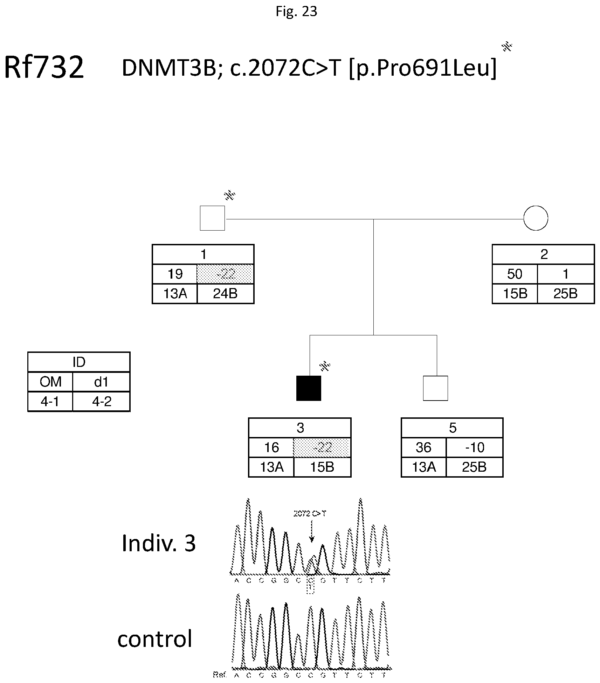

Figure 23 Pedigree of FSHD2 family Rf732 showing segregation of the DNMT3B P691L mutation (indicated with asterisk) with D4Z4 hypomethylation and disease presentation in individual 3. Both father (1) and son (3) show severe hypomethylation of the D4Z4 repeat array. Key: ID = identifier, OM = D4Z4 methylation, dl = Delta1 (difference between expected D4Z4 methylation and observed D4Z4 methylation) and the sizes of the D4Z4 repeat arrays on both chromosomes 4 (4-1 and 4-2) in units. Below: sequence track of the DNMT3B mutation in individual 3 and in a control as reference.

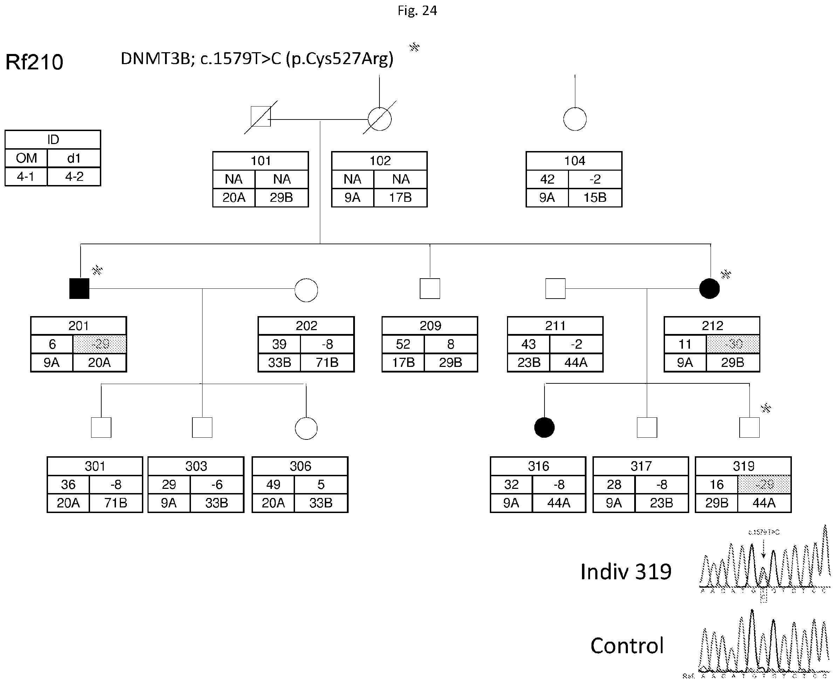

Figure 24 Pedigree of FSHD1 + FSHD2 family Rf201 showing segregation of the DNMT3B C527R mutation (indicated with asterisk) with D4Z4 hypomethylation and disease presentation in individuals 201, 212 and 319. Seven individuals carry a D4Z4 repeat array of 9 repeat units (9A), classifying this family as FSHD1. Carriers of the DNMT3B C527R show profound D4Z4 hypomethylation and in two cases, disease presentation. Key: ID = identifier, OM = D4Z4 methylation, d1 = Delta1 (difference between expected D4Z4 methylation and observed D4Z4 methylation) and the sizes of the D4Z4 repeat arrays on both chromosomes 4 (4-1 and 4-2) in units. Below: sequence track of the DNMT3B mutation in individual 319 and in a control as reference.

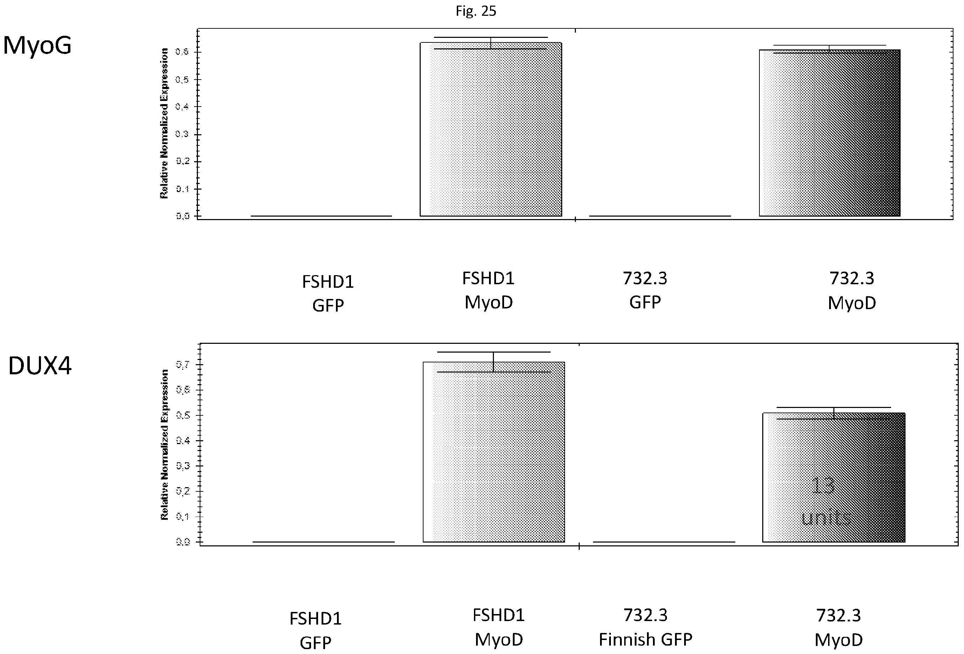

Figure 25 DUX4 expression in FSHD2 individual 732.3. Fibroblasts were forced to differentiate into myotubes by lentiviral MyoD expression. qRT-PCR shows that forced myogenesis of FSHD1 fibroblasts (as a control) and of DNMT3B mutation carrier 732.3 (FSHD2) causes DUX4 expression, the molecular hallmark of FSHD. Forced myogenesis was successful as evidenced by the expression of MyoG. As a control for myogenesis, fibroblasts were transduced with GFP.

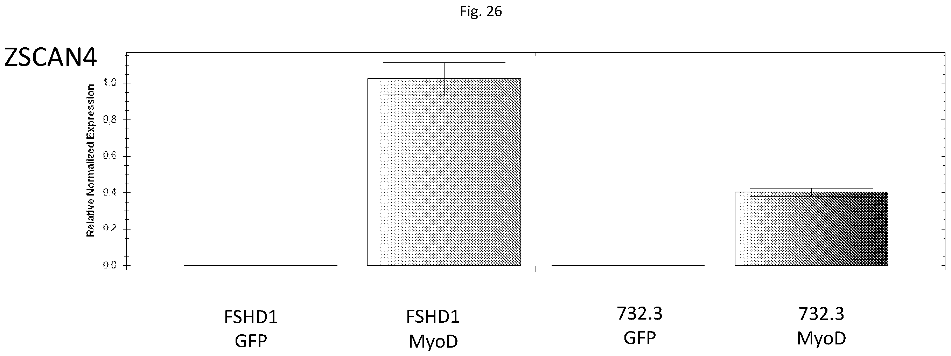

Figure 26 DUX4 expression in FSHD2 individual 732.3. Fibroblast were forced to differentiate into myotubes by lentiviral MyoD expression. qRT-PCR shows that forced myogenesis of FSHD1 fibroblasts (as a control) and of DNMT3B mutation carrier 732.3 (FSHD2) causes ZSCAN4 expression, a known target of DUX4. As a control for myogenesis, fibroblasts were transduced with GFP.

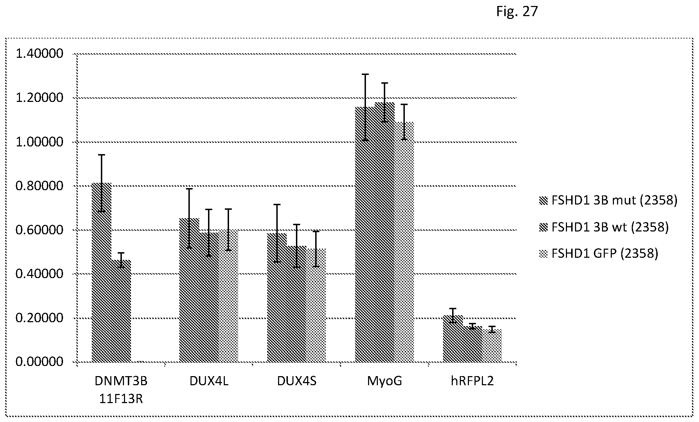

Figure 27 Expression of mutant (mut; C527R) or wildtype DNMT3B (wt) do not affect DUX4 expression levels in FSHD myotubes. qRT-PCR of expression levels of DNMT3B (11F13R), DUX4 long isoform (DUX4L), DUX4 short isoform (DUX4S), myogenin (MYOG) and the DUX4 target (hRFLP2) after transduction of lentivirusses expressing FSHD2 mutant DNTM3B (3B mut; blue), wild type DNMT3B (3B wt, red) or GFP as control (green), demonstrating that overexpression of wild type or mutant DNMT3B do not affect DUX4 expression levels.

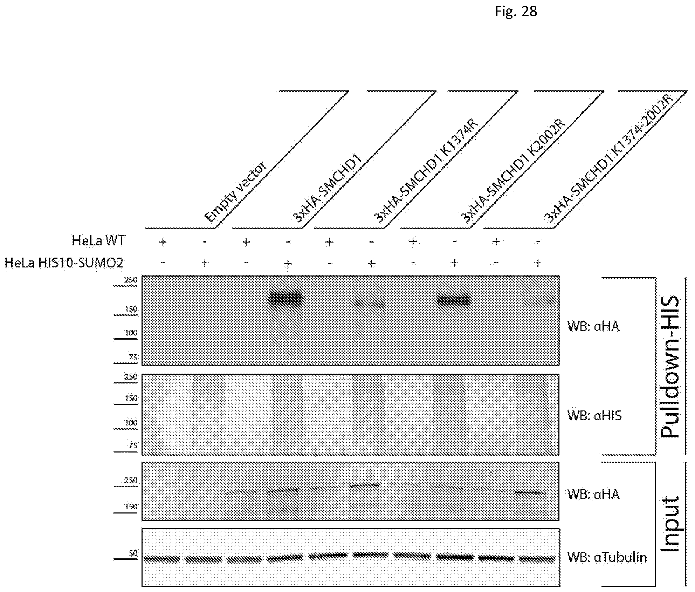

Figure 28 Western blot analysis showing immunoprecipitated SMCHD1 protein in HIS₁₀-SUMO2 HeLa lysate. Stable HeLa cell lines containing HIS₁₀-SUMO2 were transfected with wild-type or mutant HA tagged SMCHD1 expression constructs. After lysis of the cells, immunoprecipitation was performed using Ni-NTA agarose beads, which specifically and robustly bind HIS tagged proteins and the immunoprecipitated SMCHD1 protein was detected by Western blot analysis using HA antibodies. A protein of 200 kDa in size was detected, corresponding to the expected size of the SMCHD1 protein in the IP of HIS₁₀-SUMO2 HeLa lysate.
Figure 29 Selection of guide RNAs for targeting the novel SMCHD 1 exon in order to corrupt the exon-intron structure leading to exon skipping of the extra exon and restoration of the SMCHD1 reading frame.
Table S1: overview of patient derived muscle cell cultures and their experimental use.
Table S2: overview of primer sequences and shRNA constructs used in the study.
Table S3: overview of further shRNA constructs used in the study.

### DETAILED DESCRIPTION OF THE INVENTION

Unless otherwise defined, all technical and scientific terms used herein have the same meaning as commonly understood by one of ordinary skill in the art to which this invention belongs. Methods and materials are described herein for use in the present invention. However other suitable methods and materials known in the art can also be used. The materials, methods, and examples are illustrative only and not intended to be limiting, unless so indicated. In case of conflict, the present specification, including definitions, will control. The following definitions are used unless otherwise described.

As used herein, the term "mutated", employed in combination with SMCHD1, refers to loss-of-function mutations in SMCHD1 that cause FSHD2. The mutations may be small-scale mutations, such as those affecting one or a few nucleotides. These mutations may be point mutations in which a single nucleotide is exchanged for another. The point mutation occurs within the protein coding region of the SMCHD1 gene and is either a missense mutation, resulting in a codon which codes for a different amino acid, or a nonsense mutation, resulting in a stop codon which leads to a truncated SMCHD1 protein. The mutations may alternatively be insertions which add one or more extra nucleotides into the DNA. Insertions in the coding region of a gene may alter splicing of the mRNA (splice site mutation), or cause a shift in the reading frame (frameshift), both of which can significantly alter the gene product. The mutations may also be deletions which remove one or more nucleotides from the DNA, or the entire SMCHD1 gene. These mutations can also alter the reading frame of the gene.

As used herein, "exon-skipping", refers to the exclusion of a specific nucleic acid sequence (normally an exon) from SMCHD1 encoding mRNA. Exon-skipping is typically induced by an oligonucleotide that is directed toward the splice sites flanking the exon or by an exon-internal oligonucleotide. The number of bases that are sufficient and/or optimal for inducing exon skipping is dependent on several factors. Some types of oligonucleotide usually require somewhat longer oligonucleotides. For instance, morpholinos typically have between about 25-30 consecutive bases whereas 2'O-methyl phosphorothioate oligonucleotides typically have their activity optimum between about 20-25 bases. For the present invention it is therefore preferred that said anti-sense oligonucleotide is complementary to a consecutive part of between about 15 and 35 nucleotides. More preferably said oligonucleotide is complementary to a consecutive part of between about 20 and 30 nucleotides.

Different types of nucleic acid may be used to generate the oligonucleotide. Preferably, the oligonucleotide comprises RNA, as RNA/RNA hybrids are very stable. Since one of the aims of the exon skipping technique is to direct splicing in subjects it is preferred that the oligonucleotide RNA comprises a modification providing the RNA with an additional property. Some types of anti-sense oligonucleotides render the target (pre-mRNA) sensitive to nucleases such as RNAseH. Since in this case only exon-skipping is intended, it is preferred that the used anti-sense oligonucleotide does not have this property and thus comprises a modification that renders the duplex formed by the antisense oligonucleotide and the target pre-mRNA resistant to RNAseH. Various oligonucleotides and modifications and variants thereof do not promote the nuclease degradation of the target (pre-) mRNA. Examples of such types of oligonucleotides are 2'O-methyl phosphorothioate oligonucleotides and morpholinos. Other features that may be added to or enhanced in said anti-sense oligonucleotide are increased stability (for instance in a bodily fluid), increased or decreased flexibility, reduced toxicity, increased intracellular transport, tissue-specificity, etc.

An oligonucleotide of the invention may be provided to a cell as such or in the context of a nucleic acid delivery vehicle. Oligonucleotides can be transfected into the cell using a transfection agent such as a liposome or PEI. Alternatively, the oligonucleotide is introduced into the cell with a viral, or virus-based gene delivery vehicle. The oligonucleotide can also be encoded by an expression cassette provided to said cell. Typically, the oligonucleotide is encoded by an expression cassette in said viral or virus-based gene delivery vehicle. The oligonucleotide may then be contained with a transcript comprising said oligonucleotide. Thus preferably said expression cassette encodes an RNA comprising said anti-sense oligonucleotide. The gene delivery vehicle can contain an expression cassette. An expression cassette contains the sequence to be expressed together with all sequences necessary for expression of the sequence for instance a suitable promoter and transcription termination sequence. The expression vector is preferably introduced into the cell via a gene delivery vehicle. A preferred delivery vehicle is a viral vector such as an adenoviral vector and more preferably an adeno-associated virus vector.

The term "pharmaceutically acceptable carrier" as used herein refers to a carrier for administration of the active substance. The pharmaceutically acceptable carrier can comprise any substance or vehicle suitable for delivering the substance to a therapeutic target, such as muscle cells or progenitor muscle cells, and in particularly skeletal muscle cells, of the subject. The term refers to any pharmaceutical carrier that may be administered without undue toxicity. Suitable carriers may be one or more optional stabilizers, diluents, or excipients.

The term "therapeutically effective amount" as used herein refers to the quantity of the SMCHD1 protein or nucleic acid according to the invention necessary to reduce or suppress DUX4 in skeletal muscle or to prevent, cure or at least partially arrest a symptom of FSHD and/or its complications. Amounts effective to achieve this goal will, of course, depend on the type and severity of the disease and the general condition, particularly the weight, of the patient.

SMCHD1 protein is a protein of SEQ ID NO: 1 or an orthologue thereof of another species, preferably another mammal SMCHD1 protein is also an amino acid sequence of binding to a chromosome 4q35 D4Z4 repeat array in FSHD cells of a subject that has FSHD.

The level of a SMCHD1 protein is increased in a cell when the amount of SMCHD1 protein in the cell is increased by at least 10%, preferably at least 20%, more preferably at least 30, 40, 50% or more when compared to the amount of SMCHD1 protein in the cell prior to said increase. The activity of SMCHD1 is increased in a cell when the activity of SMCHD1 is increased by at least 10%, preferably at least 20%, more preferably at least 30, 40, 50% or more when compared to the activity of SMCHD1 in the cell prior to said increase. SMCHD1 activity in a cell can be increased with or without a concomitant increase in the level of SMCHD1. A preferred but not limiting way of increasing the activity of SMCHD1 in a cell is by increasing the amount of SUMOylation of SMCHD 1 in the cell.

The level and/or activity of SMCHD1 in a cell is preferably increased by providing the cell with a nucleic acid that codes for an SMCHD1 protein (coding region). The nucleic acid is preferably embedded in an expression cassette that directs the expression of the coding region. The coding region can be an SMCHD1 cDNA or a modified sequence comprising one or more (artificial) introns.

FSHD cells are cells of an individual that has FSHD1 or FSHD2. Also included are cells wherein an FSHD mutation has been introduced. In a preferred embodiment the FSHD cells are skeletal muscle cells.

The target cells for modification according to the invention are preferably muscle cells or precursors thereof. There are various precursors of muscle cells. Typical precursor cells are for example myoblast cells and (myo)satellite cells. The formation of muscular tissue is also called myogenesis. Muscle fibers form from the fusion of myoblasts into multinucleated fibers. Satellite cells are described as quiescent myoblasts. Satellite cells enable the repair of muscle. They have the ability to replicate, albeit limited. Primary muscle fibers form from primary myoblasts. Secondary muscle fibers form secondary myoblasts. The muscle fibers that form later arise from satellite cells.

Myosatellite cells or satellite cells are small mononuclear progenitor cells with virtually no cytoplasm found in mature muscle. Satellite cells are precursors to skeletal muscle cells, able to give rise to satellite cells or differentiated skeletal muscle cells, although other cells in the skeletal muscle also have this capacity such as pericytes. They have the potential to provide additional myonuclei to their parent muscle fiber, or return to a quiescent state. Upon activation, satellite cells can re-enter the cell cycle to proliferate and differentiate into myoblasts.

The substance as used herein preferably increases a SMCHD1 protein level by enhancing expression of the SMCHD1 gene in FSHD cells of the subject.

Various viral vector systems are available for transferring nucleic acid into cells. It is preferred that the cells are stably transformed by the vector. Such stable transformation is typically achieved by retroviral and lentiviral vectors that integrate into the genome of a target cell. However, stable transfer is also possible with other vectors, for instance adeno-associated virus vectors or (minimal) adenovirus vectors typically in tissue with a limited cell turnover, such as nerve cells, muscle cells and the like.

Retrovirus or retroviral vectors.

Retroviruses are one of the mainstays of current gene therapy approaches. Recombinant retroviruses such as the Moloney murine leukemia virus have the ability to integrate into the host genome in a stable fashion. They contain a reverse transcriptase that allows integration into the host genome. They have been used in a number of FDA-approved clinical trials such as the SCID-X1 trial. Retroviral vectors can either be replication-competent or replication-defective. Replication-defective vectors are the most common choice in studies because the viruses have had the coding regions for the genes necessary for additional rounds of virion replication and packaging replaced with other genes, or deleted. These virus are capable of infecting their target cells and delivering their viral payload, but then fail to continue the typical lytic pathway that leads to cell lysis and death.

Depending on the viral vector, the typical maximum length of an allowable DNA insert in a replication-defective viral vector is usually about 8 -10 kB. This limit accommodates most cDNA sequences and small RNA sequences. Retroviruses such as the Moloney retrovirus require cells to be actively dividing for transduction. As a result, cells such as neurons are resistant to infection and transduction by normal retroviruses.

Lentiviruses are a subclass of Retroviruses. They have recently been adapted as gene delivery vehicles (vectors) thanks to their ability to integrate into the genome of non-dividing cells, which is the unique feature of Lentiviruses as other Retroviruses can infect only dividing cells. The viral genome in the form of RNA is reverse-transcribed when the virus enters the cell to produce DNA, which is then inserted into the genome at a random position (recent findings actually suggest that the insertion of viral DNA is not random but directed to specific active genes and related to genome organisation) by the viral integrase enzyme. The vector, now called a provirus, remains in the genome and is passed on to the progeny of the cell when it divides. The site of integration is unpredictable, which can pose a problem. The provirus can disturb the function of cellular genes and lead to activation of oncogenes promoting the development of cancer, which raises concerns for possible applications of lentiviruses in gene therapy. However, studies have shown that lentivirus vectors have a lower tendency to integrate in places that potentially cause cancer than gamma-retroviral vectors. More specifically, one study found that lentiviral vectors did not cause either an increase in tumor incidence or an earlier onset of tumors in a mouse strain with a much higher incidence of tumors. Moreover, clinical trials that utilized lentiviral vectors to deliver gene therapy for the treatment of HIV experienced no increase in mutagenic or oncologic events was observed.

To produce a lentivirus, several plasmids are transfected into a so-called packaging cell line, commonly HEK 293. One or more plasmids, generally referred to as packaging plasmids, encode the virion proteins, such as the capsid and the reverse transcriptase. Another plasmid contains the genetic material to be delivered by the vector. It is transcribed to produce the single-stranded RNA viral genome and is marked by the presence of the ψ (psi) sequence. This sequence is used to package the genome into the virion.

As opposed to lentiviruses, adenoviral DNA does not integrate into the genome and is not replicated during cell division. Their primary applications are in gene therapy and vaccination. Since humans commonly come in contact with adenoviruses, which cause respiratory, gastrointestinal and eye infections, majority of patients have already developed neutralizing antibodies which can inactivate the virus before it can reach the target cell. To overcome this problem scientists are currently investigating adenoviruses that infect different species to which humans do not have immunity.

Adeno-associated virus (AAV) is a small virus that infects humans and some other primate species. AAV is not currently known to cause disease and consequently the virus causes a very mild immune response. AAV can infect both dividing and non-dividing cells and may incorporate its genome into that of the host cell. These features make AAV a very attractive candidate for creating viral vectors for gene therapy.

Furthermore, because of its potential use as a gene therapy vector, researchers have created an altered AAV called Self-complementary adeno-associated virus (scAAV). Whereas AAV packages a single strand of DNA and requires the process of second-strand synthesis, scAAV packages both strands which anneal together to form double stranded DNA. By skipping second strand synthesis scAAV allows for rapid expression in the cell. Otherwise, scAAV carries many characteristics of its AAV counterpart.

Nonviral substances such as Ormosil have been used as DNA vectors and can deliver DNA loads to specifically targeted cells in living animals. (Ormosil stands for organically modified silica or silicate.)

The subject in the present invention is preferably a mammal subject, preferably a primate and most preferably a human.

Small RNAs are a type of non-coding RNA (ncRNA) molecule that are 50-250 nucleotides in length. They are often involved in regulating the translation of target RNAs through RNA/RNA interactions.

A non-coding RNA (ncRNA) is a functional RNA molecule that is not translated into a protein. Less-frequently used synonyms are non-protein-coding RNA (npcRNA), non-messenger RNA (nmRNA) and functional RNA (fRNA). The DNA sequence from which a non-coding RNA is transcribed is often called an RNA gene. Non-coding RNA genes include highly abundant and functionally important RNAs such as transfer RNAs (tRNAs) and ribosomal RNAs (rRNAs). For the present invention the term small RNA is reserved for non-coding RNA that is not a tRNA and not a rRNA. Also not included in the definition are the long non-coding RNA as Xist and HOTAIR. Also not included are the spliceosomes RNAs U1, U2 etc. Non-coding RNAs do include RNAs such as snoRNAs, microRNAs, siRNAs, snRNAs, exRNAs, and piRNAs. The number of ncRNAs encoded within the human genome is unknown; however, recent transcriptomic and bioinformatic studies suggest the existence of thousands of ncRNAs.

SMCHD1 peptides were found in a mass spec screen for SUMO-2 target proteins purified from cells upon knockdown of the SUMO-targeted ubiquitin ligase RNF4. SUMOylated SMCHD1 is ubiquitinated by Ring Finger Protein 4 (RNF4), encoded by the RNF4 gene.

RNA interference (RNAi) is a biological process in which RNA molecules inhibit gene expression, typically by causing the destruction of specific mRNA molecules. Historically, it was known by other names, including co-suppression, post transcriptional gene silencing (PTGS), and quelling. Only after these apparently unrelated processes were fully understood did it become clear that they all described the RNAi phenomenon.

The phenomenon of RNA interference (RNAi) is involved in sequence-specific gene regulation driven by the introduction of dsRNA resulting in inhibition of translation or transcriptional repression. Two types of small ribonucleic acid (RNA) molecules - microRNA (miRNA) and small interfering RNA (siRNA) - are central to RNA interference. RNAs are the direct products of genes, and these small RNAs can bind to other specific messenger RNA (mRNA) molecules and either increase or decrease their activity, for example by preventing an mRNA from producing a protein. RNA interference has an important role in defending cells against parasitic nucleotide sequences - viruses and transposons. It also influences development.

The RNAi pathway is found in many eukaryotes, including animals, and is initiated by the enzyme Dicer, which cleaves long double-stranded RNA (dsRNA) molecules into short double stranded fragments of ~20 nucleotide siRNAs. Each siRNA is unwound into two single-stranded RNAs (ssRNAs), the passenger strand and the guide strand. The passenger strand is degraded and the guide strand is incorporated into the RNA-induced silencing complex (RISC). The most well-studied outcome is post-transcriptional gene silencing, which occurs when the guide strand pairs with a complementary sequence in a messenger RNA molecule and induces cleavage by Argonaute, the catalytic component of the RISC complex. In some organisms, this process spreads systemically, despite the initially limited molar concentrations of siRNA

An antisense molecule typically comprises 15-40 nucleotides of which at least 15 consecutive nucleotides are complementary to and hybridize with a pre-mRNA of a target gene. The antisense molecule is preferably an antisense oligonucleotide or AON. The AON may be RNA, DNA or a combination thereof. Typically but not necessarily the AON comprises a modified backbone, a modified base or a combination thereof. Many different chemistries exist. To allow for instance exon-skipping or polyA interference an AON typically has a chemical modification that renders the AON/RNA duplex insensitive to the action of RNAseH. Various chemical modifications are for instance described in Annemieke Aartsma-Rus and Gert-Jan B. van Ommen RNA. 2007 Oct; 13(10): 1609-1624. "Antisense-mediated exon skipping: A versatile tool with therapeutic and research applications"

A small hairpin RNA or short hairpin RNA (shRNA) is an artificial RNA molecule with a tight hairpin turn that can be used to silence target gene expression via RNA interference (RNAi). Expression of shRNA in cells is typically accomplished by delivery of plasmids or through viral or bacterial vectors. shRNA is an advantageous mediator of RNAi in that it has a relatively low rate of degradation and turnover. However, it requires use of an expression vector. The promoter is chosen to achieve robust shRNA expression. Polymerase III promoters such as U6 and H1 can be used. When control over the expression is desired polymerase II promoters can be used to regulate shRNA expression.

### EXAMPLES

The invention will now be illustrated by means of the following examples, which examples are provided by way of illustration and not of limitation. It will be understood that many variations in the methods described can be made

### Example 1 - DUX4 expression is induced upon myogenic differentiation and coincides with reduced SMCHD1 levels.

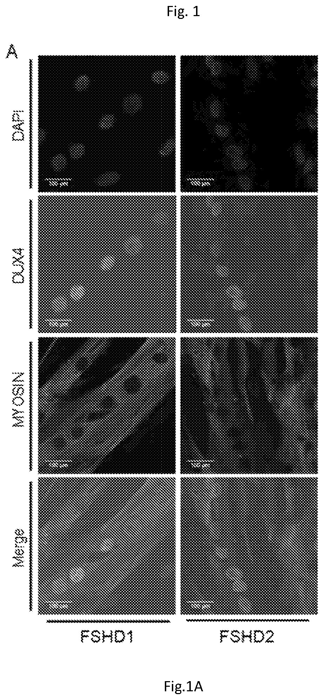

Although DUX4 expression has been reported repeatedly in FSHD myotubes (8,25-27), comprehensive data on the regulation of DUX4 expression in FSHD1 and FSHD2 muscle cells is scarce. Therefore, a first aim was to firmly establish that DUX4 expression increases with differentiation of FSHD1 and FSHD2 muscle cells. A set of primary myoblasts derived from control, FSHD1 and FSHD2 individuals was cultured and RNA from both proliferating myoblasts and differentiating myotubes (further referred to as myoblasts and myotubes) was harvested. The differentiation status of the cultures was confirmed by myotube formation, as visualized by immunofluorescent labeling of myosin, and MYOG levels (Fig. 1A, Fig 5 and 6).

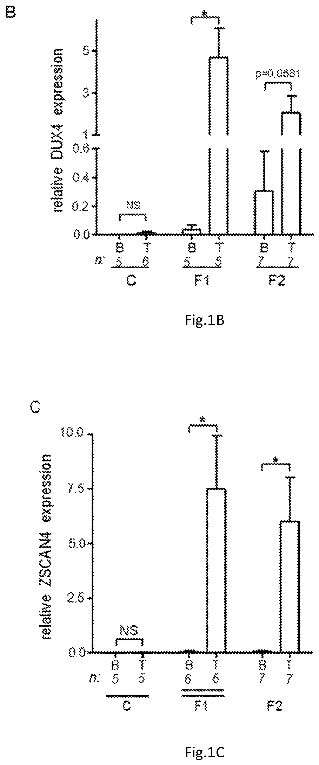

By mRNA quantification using qRT-PCR it is confirmed that *DUX4* in FSHD1 and FSHD2 myotubes is activated, whereas DUX4 transcripts in control samples could not reproducibly be detected (Fig. 1B). Immunofluorescence microscopy on a subset of myotube cultures established the variegated pattern of DUX4 protein expression in both FSHD1 and FSHD2 (Fig. 1A and Fig. 6). Expression levels of the previously established DUX4 target gene ZSCAN4 were also quantified and these levels support the FSHD-specific activation during myoblast differentiation (Fig. 1C) (9).

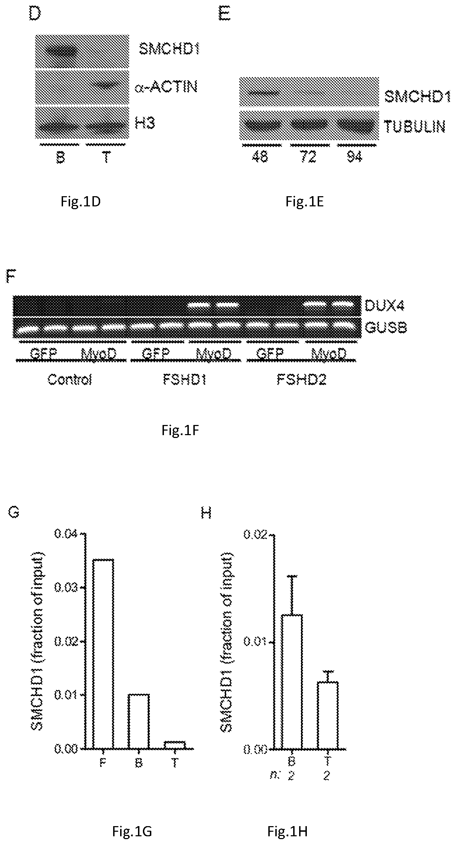

Since it was previously demonstrated that SMCHD1 is a D4Z4 chromatin repressor (14), SMCHD1 protein levels were examined. Western blot analysis shows a clear reduction in SMCHD1 protein levels after myoblast differentiation (Fig. 1D), a phenomenon that could be reproduced by forced myogenic differentiation of control fibroblasts transduced with the myogenic transcription factor MyoD1 (Fig. 1E). Upon forced myogenesis in FSHD fibroblast cell lines activation of DUX4 is observed, whereas DUX4 remains undetectable in control samples treated with MyoD1 (Fig. IF). A higher density of SMCHD1 at qD4Z4, located around the transcriptional start site of DUX4 is observed in fibroblasts as compared to myoblasts when employing ChIP-qPCR in control fibroblast, myoblast and myotube cultures from the same donor (Fig. 6) (21), with a further decrease in myotubes (Fig. 1G). Analysis of two additional independent control myoblast and myotube cultures confirmed the decrease in SMCHD1 levels at D4Z4 after myoblast differentiation (Fig. 1H). Together, this confirms the FSHD specific induction of DUX4 expression upon myogenic differentiation in a large set of FSHD1 and FSHD2 primary myotube cultures and upon MyoD-induced differentiation in FSHD fibroblasts. Moreover, this demonstrates that DUX4 expression correlates with a reduction in SMCHD1 levels at D4Z4 in myogenic cell lines, specifically in differentiated myotubes.

### Example 2 - SMCHD1 levels control DUX4 expression in somatic cells.

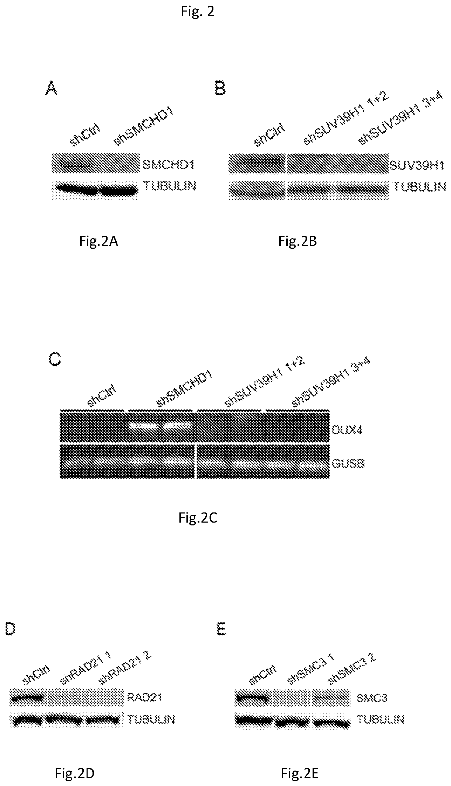

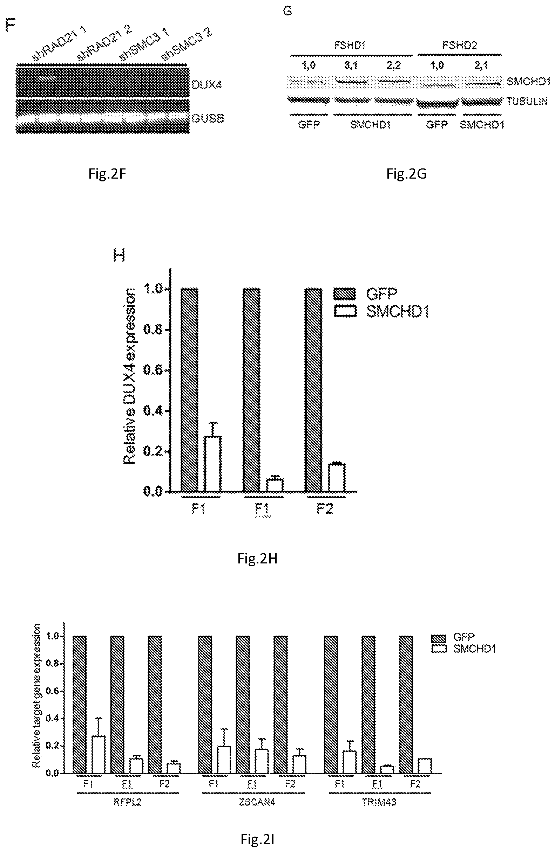

To establish a causal relationship between DUX4 expression in myotubes and SMCHD1 protein levels, lentiviral shRNA-mediated depletion experiments were conducted in a differentiating primary control myoblast culture, carrying an FSHD-permissive (DUX4-PAS containing) 4A161 allele. Depletion of SMCHD1 resulted in robust activation of DUX4 expression (Fig. 2A, C), which is in agreement with earlier observations [14]. This phenomenon of DUX4 activation seems to be unique for SMCHD1 since knocking down other known chromatin repressors of D4Z4 in parallel experiments using the same control myoblast culture did not lead to DUX4 expression. Upon knockdown of SUV39H1, the histone methyltransferase shown to be involved in establishing the H3K9me3 modification at D4Z4 [21], a 20% reduction in relative abundance of H3K9me3 at D4Z4 is observed (Fig. 2B and Fig. 8). SUV39H1 knockdown, however, does not lead to transcriptional activation of DUX4 (Fig. 2B-C). Similarly, independent depletion of cohesin proteins SMC3 or RAD21 by a similar strategy does not cause a consistent activation of DUX4 either (Fig. 2E-F).

Since SMCHD1 levels naturally decline at D4Z4 during myoblast differentiation, it is tested whether ectopic expression of SMCHD1 in FSHD myotubes rescues the repression of DUX4. To this end, FSHD1 and FSHD2 myoblast cultures were transduced with SMCHD1 expressing lentiviruses and the cells were allowed to form myotubes. In both FSHD1 and FSHD2 myotubes a 2-3 fold overexpression of SMCHD1, as determined by western blot analyses, resulted in a 70-90% reduction in DUX4 mRNA levels (Fig. 2G-H). To confirm the silencing of DUX4, the mRNA expression levels of three known downstream target genes of DUX4 protein were analysed. ZSCAN4, RFPL2, and TRIM43 expression levels decreased 70-90% upon overexpression of SMCHD1 (Fig. 2I). To rule out that the lower DUX4 transcript levels were a consequence of reduced muscle cell differentiation, MYOG expression was also measured. Although variable, MYOG mRNA levels were not decreased upon SMCHD1 overexpression (Fig. 9). In conclusion, DUX4 expression levels are strongly decreased upon moderate overexpression of SMCHD1, emphasizing its role as D4Z4 repressor in both genetic forms of the disease.

### Example 3 - SMCHD1 reduction leads to PRC2 enrichment and CpG hypomethylation at D4Z4.

Since SMCHD1 was recently shown to associate with the repressive H3K27me3 modification at the inactive X chromosome (29), the levels of H3K27me3 and the PRC2 complex at D4Z4 upon depletion of SMCHD1 in control myotubes were determined. SMCHD1 knockdown resulted in a 2-fold decrease in SMCHD1 levels at D4Z4 (Fig. 3A). Using two different SMCHD1 shRNA vectors some evidence for decreased CpG methylation at D4Z4 (Fig. 3B) and increased H3K27me3 levels at D4Z4 upon SMCHD1 depletion (Fig. 3C) was observed, but none of these trends met the standard threshold for statistical significance. Because of the increase in H3K27me3 at D4Z4 upon SMCHD1 depletion, the involvement of the PRC2 complex was further analyzed and a strong increase of the PRC2 protein SUZ12 at D4Z4 was observed (Fig. 3D).

To validate these data derived from knock down experiments in a single control myotube culture, the levels of CpG methylation and the PRC2 complex in primary FSHD myotubes were investigated. CpG methylation levels showed a significant decrease in FSHD2 derived samples as compared to controls. FSHD1 derived samples showed intermediate levels (Fig. 3E), as reported for other tissues (18). Previously, H3K27me3 levels at D4Z4 were only tested in proliferating control and FSHD1 myoblast cultures and reported to be indifferent at the DBE locus just proximal to qD4Z4 (Fig. 7) (17). H3K27me3 levels at qD4Z4 in FSHD and control myotube cultures were measured and significantly higher levels of H3K27me3 in FSHD2 myotubes were observed (Fig. 3F), consistent with the increase observed following knock-down of SMCHD1 (Fig. 3C). Increased H3K27me3 levels were also observed in FSHD2 myoblasts (Fig. 10). Next, the levels of the PRC2 proteins SUZ12 and EZH2 at D4Z4 were quantified in a smaller set of samples. Higher levels of both proteins at D4Z4 were detected in FSHD2 samples (Fig. 3G-H), all consistent with an increase in H3K27me3 through PRC2 recruitment at D4Z4.

### Example 4 - Some D4Z4 chromatin changes support a role for myogenic differentiation in DUX4 activation.

Since DUX4 expression could not be activated upon knock down of cohesin or SUV39H1, other known D4Z4 histone modifications in the context of myogenic differentiation were reevaluated. The relative abundance of H3K9me3, which was previously shown to be deposited at D4Z4 by SUV39H1, was quantified. A trend to lower levels of H3K9me3 in FSHD2 myoblasts was observed (Fig. 4A), but in myotubes a difference between controls and FSHD in the relative abundance of this histone modification was not observed. H3K9me3 at D4Z4 is bound by HP1γ, which in turn recruits the multi subunit cohesin complex to this locus, contributing to the repressive chromatin structure [21]. The cohesin complex, consisting of SMC1, SMC3, RAD21 and SA1/2, was detected at D4Z4 in HeLa cells, fibroblasts and myoblasts, but not in lymphoblastoid cell lines [21]. Cohesin levels in control and FSHD myoblasts and myotubes were quantified by means of RAD21 ChIP-qPCR. The reduced chromatin compaction in FSHD myoblasts was indeed reflected by reduced levels of RAD21 at qD4Z4 (Fig. 4B) compared to control samples, as was shown previously in fibroblasts [21]. However, upon differentiation a significant reduction of RAD21 in control myotubes was observed, resulting in comparable levels of RAD21 in control and FSHD myotubes (Fig. 4B). To further corroborate this observation, the relative abundance of another cohesin component, SMC3, at qD4Z4 was determined in a control sample and a similar reduction upon differentiation was observed (Fig. 11).

In conclusion, the levels of repressive chromatin features H3K9me3 and cohesin at D4Z4 do not correlate with DUX4 activation during muscle cell differentiation.

In general, chromatin at promoters of actively transcribed genes is marked by H3K4me2 and H3K4me3, whereas the gene body is enriched for H3K36me3 (30). Despite the sporadic nature of DUX4 expression in only a small number of FSHD myonuclei, nevertheless evidence was found for chromatin changes at the 5' end of *DUX4* by ChIP-qPCR (qD4Z4; Fig. 7) correlating with *DUX4* transcriptional activity in FSHD myotubes. H3K4me2, marking active promoters, was found at qD4Z4 and showed a significant increase in FSHD2 myoblasts and a similar trend in myotubes (Fig. 4C). H3K4me3, peaking at transcriptional start sites (TSS) of active genes, also showed a trend towards an increase in FSHD2 myotubes (Fig. 4D), consistent with the location of the qD4Z4 amplicon. Increases in H3K4me2 and H3K4me3 were not so evident in FSHD1 myoblasts and myotubes possibly because in FSHD2 cells the chromatin structure of all D4Z4 repeat arrays are affected while in FSHD1 the effect is diluted by the presence of three normal D4Z4 repeat arrays (18). Transcriptional activity at this site was furthermore reflected by a significant increase of H3K36me3 upon differentiation in FSHD myoblast cultures (Fig. 4E). Thus, despite the repetitive nature of D4Z4 arrays and the sporadic activation of *DUX4,* we found evidence for chromatin changes correlating with *DUX4* transcription in FSHD myotubes, but not in myoblasts or control myotubes.

### Example 5 - Sumoylation of SMCHD1 stimulates repression of DUX4.

Posttranslational modification of lysine residues in proteins by small ubiquitin-like modifiers (SUMO) is a reversible and dynamic process that regulates protein localization, stability and protein-protein interactions. SMCHD1 was identified as a SUMOylated protein by SUMO1 and SUMO2 in several different studies, by using genome wide mass spectrometry approaches. Recently, specific sumoylation sites were also identified for SMCHD1. Using immunoprecipitation (IP) it is confirmed that SMCHD1 is sumoylated by SUMO1 and SUMO2 (Fig. 12).

Small Ubiquitin-like Modifier (or SUMO) proteins are a family of small proteins that are covalently attached to and detached from other proteins in cells to modify their function. Sumoylation is a post-translational modification involved in various cellular processes, such as nuclear-cytosolic transport, transcriptional regulation, apoptosis, protein stability, response to stress, and progression through the cell cycle. SUMO proteins are similar to ubiquitin, and sumoylation is directed by an enzymatic cascade analogous to that involved in ubiquitination. In contrast to ubiquitin, SUMO is not used to tag proteins for degradation. Mature SUMO is produced when the last four amino acids of the C-terminus have been cleaved off to allow formation of an isopeptide bond between the C-terminal glycine residue of SUMO and an acceptor lysine on the target protein. Sumoylation is a dynamic process. A complex enzyme cascade has been identified to couple the SUMO substrates to proteins and also to remove the substrates. It was hypothesized that based on published studies sumoylation is necessary for optimal activity of proteins. Hence it was speculated that optimal repressor activity of the chromatin repressor requires protein sumoylation. Consequently, it was investigated whether inhibiting the coupling of the SUMO substrate by depleting UBA2 E3 ligase, a component of the sumoylation cascade, leads to DUX4 expression in control human primary myoblasts (Fig. 13). It is shown that downregulation of UBA2 using an shRNA as indicated in table S3 in primary control muscle cell cultures leads to expression of the normally repressed DUX4 suggesting that intact sumoylation (of SMCHD1) is necessary for repression of DUX4 transcription.

To further confirm the hypothesis that sumoylation is relevant for repression of DUX4, PR619 was used as inhibitor for the removal of SUMO substrates from their proteins. PR619 is a non-selective inhibitor of deubiquitinases and ubiquitin-like isopeptidases (like the family of SENP isopeptidases). The hypothesis was that the inhibition of removal of SUMO substrate will increase the repressor activity of SMCHD1 and hence will lead to suppression of DUX4 expression. DUX4 expressing human primary FSHD myoblasts were treated with 3 different concentrations of PR619 (5 µM, 10 µM and 20 µM) and cells were harvested for RNA isolation. It was observed that DUX4 transcript levels were decreased in cells treated with PR-619 in a dose dependent manner (Fig.14A). Thus PR619 treatment of DUX4 expressing primary FSHD myotubes leads to DUX4 downregulation.

Potential SUMOylation sites of SMCHD1 were identified at positions K1374 and K2002 (Hendriks et al. Nat. Comm. 2015; June 15). The following experiment was performed to confirm if these amino acids are indeed sumoylated. Briefly, the lysines at positions 1374 and 2002 were mutated into arginine, which is not a substrate for sumoylation. Immunoprecipitation was performed with His-labeled SUMO2 in order to determine the level of sumoylation for the different mutant proteins. SMCHD1 proteins mutated at K1374 or at K1374 andK2002 are present at low abundance in the IP fractions compared to wild-type SMCHD1. In contrast, the abundance of SMCHD 1 protein mutated at K2002 is comparable to wild-type SMCHD1 (Fig. 28). These results indicate that SMCHD1 is sumoylated at K1374 by SUMO2.

Additionally knock down experiments for sentrin-specific proteases (SENPs) further demonstrate that sumoylated SMCHD1 leads to DUX4 downregulation. The activity of both SENP1 and SENP2 was modulated in DUX4 expressing primary FSHD myoblast to observe the effect of direct interference with sumoylation on DUX4 expression. Results show that depletion of SENP1 does not lead to reduction in DUX4 expression level in FSHD2 myoblasts. However depletion of SENP2 drastically decreases DUX4 mRNA levels (Fig.14B). These results further support the notion that modulation of SUMOylation levels in FSHD cell cultures effect DUX4 expression levels.

### Example 6 - LRIF1 activity influences DUX4 expression.

LRIF1/HBiX1/C1orf103 was reported to interact with SMCHD1 based on semiquantitative MS data and co-immunoprecipitation studies. Immunofluorescence microscopy also showed that LRIF1/HBiX1/C1orf103 colocalizes with SMCHD1 on the inactive X chromosome in female cells. To test the possibility that LRIF1/HBiX1/C1orf103 activity regulates DUX4 expression LRIF1/HBiX1/C1orf103 was depleted by lentiviral knockdown with 2 different shRNA constructs in control, FSHD1 and FSHD2 human primary myoblasts. LRIF1/HBiX1/C1orf103 and DUX4 expression was monitored by qRT-PCR. The results show that in two independent FSHD1 and two independent FSHD2 myotube cultures 30-79% depletion of LRIF1/HBiX1/C1orf103 was found. DUX4 levels are significantly lower as compared to the cell cultures treated with scrambled (control) shRNAs (Fig. 15). Depletion of LRIF1/HBiX1/C1orf103 in a control cell line did not lead to DUX4 expression (data not shown). The results suggest that LRIF1/HBiX1/C1orf103 activity contributes to DUX4 transcription in FSHD1 and FSHD2 cells.

### Example 7 - DNMT3B is another FSHD2 gene.

Some 15% of FSHD2 cannot be explained by mutations in SMCHD1, yet these individuals are clinically identical, have a D4Z4 repeat array that is permissive for DUX4 expression (containing a DUX4 polyadenylation signal), and show profound D4Z4 hypomethylation, comparable to what is observed in SMCHD1 mutation carriers.

Some of these individuals carry a heterozygous mutation in the DNA methyltransferase 3B (DNMT3B) gene. In family Rf732, DNMT3B is the disease gene since it segregates with D4Z4 hypomethylation, and causes a clinical phenotype of FSHD in the presence of a D4Z4 repeat array of 13 units. In Rf201, DNMT3B is a disease modifier gene, since in this FSHD1 family, of the seven individuals that carry a D4Z4 repeat array of 9 units, only those that also carry a mutation in DNMT3B, are affected by FSHD. Also in this family, the mutation segregates with profound D4Z4 hypomethylation (Figs 23 and 24)

To provide further evidence that DNMT3B is a FSHD2 gene and when mutated causes DUX4 expression, in the absence of muscle cell cultures from these individuals, we transduced primary fibroblast cultures of DNMT3B mutation carrier 732.3 with a lentivirus expressing the myogenic transcription factor MyoD. Ectopic expression of MyoD transdifferentiates fibroblasts into myotubes as evidenced by the expression of MyoG. Transdifferentiation of 732.3 fibroblasts into myotubes reveals the presence of DUX4, the molecular hallmark of FSHD, and of the DUX4 target ZSCAN4, supporting the clinical diagnosis of FSHD.

### Example 8 - Ectopic expression of DNMT3B in FSHD1 cells does not rescue DUX4 repression

Loss of SMCHD1 function in muscle cells of FSHD patients causes DUX4 expression. Rescue of DUX4 suppression can be achieved by increasing SMCHD1 levels or activity, providing evidence that the epigenetic derepression in FSHD is reversible. Overexpressing wild type or mutant DNMT3B in FSHD1 myotube cultures by lentiviral transduction, however, does not affect DUX4 expression levels (Fig 25-27). Neither does expression of mutant DNMT3B further increase DUX4 expression, nor does expression of wild type DNMT3B suppress DUX4. DNMT3B is mainly expressed during early stages of development and is therefore unable to affect DUX4 expression at later stages of development, such as in muscle cells. This demonstrates that reversibility of chromatin repression, i.e. the ability of reversing the loss of repressor activity by increasing its expression levels or activity, is not universal but a unique aspect of SMCHD1.

### Materials and Methods

### Culturing of human primary myoblast cell lines.

Human primary myoblast cell lines were originating from the University of Rochester bio repository (http://www.urmc.rochester.edu/fields-center/). Muscle samples were obtained after subjects were consented under a protocol approved by the institutional review board at the University of Rochester. Myoblasts were cultured in DMEM/F-10 media (#31550 Gibco/Life Technologies, Bleiswijk, The Netherlands) supplemented with 20% heat inactivated fetal bovine serum (FBS #10270 Gibco), 1% penicillin/streptomycin (P/S) (#15140 Gibco) and 10ng/ml rhFGF (#G5071 Promega, Leiden, The Netherlands) and 1µM dexamethasone (#D2915 Sigma-Aldrich, Zwijndrecht, The Netherlands) was added to the medium. Myoblasts were fused at 80% confluency by culturing them in DMEM/F-12 Glutamax media (#31331, Gibco) containing 1% P/S and 2% KnockOut serum replacement formulation (#10828 Gibco) for 36 hours. Human control fibroblast cell lines were maintained in DMEM/F-12, supplemented with 20% FCS, 1% P/S, 10 mM HEPES and 1mM sodium pyruvate (all Gibco). Used cell lines, D4Z4 allele information and experimental use are listed in supplemental table S1.

### RNA isolation, cDNA synthesis and qRT-PCR.

Myoblast and myotube samples were harvested for RNA isolation by adding QIAzol lysis reagent (#79306 Qiagen N.V., Venlo, The Netherlands). RNA was isolated by miRNeasy Mini Kit (#217004 Qiagen) including DNase treatment according to the manufacturer's instructions. cDNA was synthesized with RevertAid H Minus First strand cDNA Synthesis Kit (#K1632 Thermo Fischer Scientific Inc., Waltham, MA) using 2µg template RNA and poly-dT primers. Gene specific cDNA products were quantified by qPCR in duplicate using SYBR green master mix supplemented with gene specific primers (table S2) using the CFX96 system (Bio-Rad, Veenendaal, The Netherlands). Data were analyzed by Bio-Rad CFX manager version 3.0 (Bio-Rad) using GAPDH and GUSB as reference genes.

### DNA isolation and methylation analysis.

DNA was isolated from cultured cells or PBLs using a standard salting out method and subjected to methylation quantification using the methylation sensitive restriction enzyme FseI (#R0588L, New England Biolabs / Bioké, Leiden, The Netherlands) gel electrophoresis and Southern blotting as described before (14).

### DNMT3B mutation analysis

DNMT3B mutation analysis by PCR amplification of the coding exons and Sanger sequencing has been described in Weemaes et al., Eur J Hum Genet 2013, 11:1219-1225

### Transduction of primary cell cultures.

Full length SMCHD1 and DNMT3B was PCR amplified from cDNA and cloned into the pRRL-CMV lentiviral backbone containing a puromycin selection marker using standard cloning procedures. DNMT3B mutations were introduced by site directed mutagenesis. All obtained constructs were verified by Sanger sequencing. shRNA constructs originating from the Mission shRNA library (MISSION shRNA library, TRC1 or TRC2; Sigma Aldrich) and are listed in supplemental table S2. Constructs were used to generate lentiviral particles, and myoblast cell lines were transduced and 24 hours after transduction they were grown in media containing 0.5 pg/ml puromycin. Differentiation to myotubes was induced by serum reduction at 80% confluency and cells were harvested after 36 hours of differentiation. Transduction of fibroblasts to ectopically express MyoD1 and induce myogenesis was performed as described previously (32).

### Chromatin Immunoprecipitation.

Histone ChIP studies were carried out as described before (33) using antibodies against H3 (ab1791, 2µl/rxn, Abcam, Cambridge, UK), H3K4me2 (39141, 4µl/rxn Active Motif, La Hulpe, Belgium), H3K4me3 (#17-614, 3 µl/rxn, Merck-Millipore, Amsterdam, The Netherlands), H3K9me3 (39161, 5 µl/rxn, Active Motif), H3k27me3 (#17-622, 5 µl/rxn, Merck-Millipore), H3K36me3 (2 µl/rxn, pAb-058-050, Diagenode, Seraing (Ougrée), Belgium) and total IgG (5 µl/rxn, Merck-Millipore). Non histone ChIPs were carried out as described before (14) using antibodies against SMCHD1 (ab31865, 5 pg/rxn, Abcam) RAD21 (ab992, 5 pg/rxn, Abcam), SMC3 (ab9263, 5 pg/rxn, Abcam), SUZ12 (D39F6, 5 µg/rxn, Cell Signaling, Leiden, The Netherlands), JARID2 (ab48137, 5 pg/rxn, Abcam) and EZH2 (D2C9, 5 pg/rxn, Cell Signaling).Western blot

Cell were directly lysed in NuPAGE LDS Sample Buffer and loaded on Novex 4-12% Bis-Tris Protein Gels (both Life technologies) according to manufacturer's instructions. Running of the gel and transfer to Hybond nitrocellulose membranes (GE Healthcare, Diegem, Belgium) were carried out using the Nupage Novex SDS-page gel system (Life technologies). Blots were blocked in 4% milk in PBS and incubated with antibodies against SMCHD1 (1:250), SUV39H1 (0.1 pg/ml,#07-958, Merck-Millipore), RAD21 (1:500), SMC3 (1:1000) and Tubulin (1:2000, T6199, Sigma-Aldrich). Detection and relative quantification were done using the Odyssey system (V3.0, LICOR Biosciences, Lincoln NE, USA), except for blots in figure 1, which were visualized by enhanced chemiluminescence (ECL).

For the purpose of clarity and a concise description, features are described herein as part of the same or separate aspects and preferred embodiments thereof, however, it will be appreciated that the scope of the invention may include embodiments having combinations of all or some of the features described.

**Table S1: overview of patient derived muscle cell cultures and their experimental use.**

| Sample: | D4Z4 alleles (chromosome 4): | Used for: |
|---|---|---|
| Control 1 | 94kb 168B, 128kb 161A | ChIP, expression |
| Control 2 # | 28kb 163B, 65kb 161A | ChIP, expression |
| Control 3 | 47kb 161A, 74kb 168B | ChIP, expression |
| Control 4 | 85kb 168B, 166kb 163B | ChIP, expression |
| Control 5 | 47kb 161A, 72kb 168B | ChIP, expression |
| Control 6 | 65kb A161, 101kb B163 | ChIP |
| Control 7 | 47kb 161A, 72kb 168B | Expression |
| FSHD1 1† | 23kb 161A, 178kb 168B | ChIP, expression |
| FSHD1 2 | 27kb 161A, 87kb 163B | ChIP |
| FSHD1 3 | 19kb 161A, 47kb 163B | ChIP, expression |
| FSHD1 4 | 27kb 161A, 90kb 162B | ChIP, expression |
| FSHD1 5 | 26kb 161A, 82kb 168B | ChIP, expression |
| FSHD1 6† | 28kb A161, 44kb H168A | Expression |
| FSHD2 1 | 43kb 161A, 132kb 163B | ChIP, expression |
| FSHD2 2^{∗} | 44kb 161A, 63kb 161A | ChIP, expression |
| FSHD2 3^{∗} | 50kb 161A, 58kb 163B | ChIP, expression |
| FSHD2 4† | 47kb 161A, 76kb 161A | ChIP, expression |
| FSHD2 5^{∗} | 44kb 161A, 121kb H 166A | ChIP, expression |
| FSHD2 6 | 90kb 161A, 131kb 166A | ChIP, expression |
| FSHD2 7 | 54kb 161A, 220kb 161A | ChIP, expression |
| FSHD2 8 | 54kb 161A, 70kb 161A | Expression |
| FSHD2 9 | 67kb 161A, 337kb 168B | Expression |
| FSHD2 10 | 65kb 161A, 160kb 161A | Expression |
| # Control sample used for knockdown experiments | | |
| t FSHD samples used for ectopic SMCHD1 expression | | |
| ^{∗} Samples selected on hypomethylation. no SMCHD1 mutation detected | | |

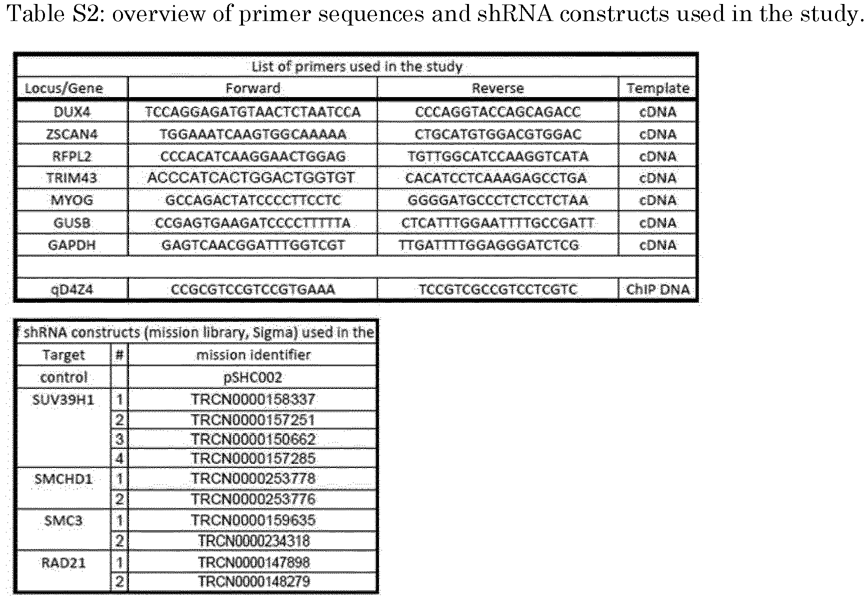

**Table S3: overview of shRNA constructs used in the study.**

| Gene name | TRC library identifier | sequence |
|---|---|---|
| UBA2 1 | TRCN0000007470 | CCGGGCTGTATTGAAAGTAGGAATACTCGAGTATTCCTACTTTCAATACAGCTTTTT |
| SENP1 1 | TRCN0000004396 | CCGGCGAGAAAGATTGCGCCAGATTCTCGAGAATCTGGCGCAATCTTTCTCGTTTTT |
| SENP12 | TRCN0000004398 | CCGGCCGAAAGACCTCAAGTGGATTCTCGAGAATCCACTTGAGGTCTTTCGGTTTTT |
| SENP2 1 | TRCN0000004577 | CCGGCGTCGCCATAGCAAAGGTAATCTCGAGATTACCTTTGCTATGGCGACGTTTTT |
| SENP2 2 | TRCN0000004579 | CCGGCCACACAAGAACAAACGCTAACTCGAGTTAGCGTTTGTTCTTGTGTGGTTTTT |
| LRIF1 1 | TRCN0000063508 | CCGGGCAGCTTATGAACAAAGTCATCTCGAGATGACTTTGTTCATAAGCTGCTTTTTG |
| LRIF1 2 | TRCN0000063509 | CCGGCCAAGATAATCTACAGCCTTTCTCGAGAAAGGCTGTAGATTATCTTGGTTTTTG |
| Control shRNA1 | pSHC004 | CCGGCGTGATCTTCACCGACAAGATCTCGAGATCTTGTCGGTGAAGATCACG TTTTT |
| Control shRNA1 | pSHC007 | CCGGCGCTGAGTACTTCGAAATGTCCTCGAGGACATTTCGAAGTACTCAGCG TTTTT |

### REFERENCES

1. Tawil,R., van der Maarel,S.M., Tapscott,S.J. (2014) Facioscapulohumeral dystrophy: the path to consensus on pathophysiology. Skelet. Muscle, 4, 12.
2. Deenen,J.C., Arnts,H., van der Maarel,S.M., Padberg,G.W., Verschuuren,J.J., Bakker,E., Weinreich,S.S., Verbeek,A.L., van Engelen,B.G. (2014) Population-based incidence and prevalence of facioscapulohumeral dystrophy. Neurology 83: 1056-1059.
3. Gabriels,J., Beckers,M.C., Ding,H., De Vriese,A., Plaisance,S., van der Maarel,S.M., Padberg,G.W., Frants,R.R., Hewitt,J.E., Collen,D., Belayew,A. (1999) Nucleotide sequence of the partially deleted D4Z4 locus in a patient with FSHD identifies a putative gene within each 3.3 kb element. Gene, 236, 25-32.
4. Wijmenga,C., Frants,R.R., Brouwer,O.F., Moerer,P., Weber,J.L., Padberg,G.W. (1990) Location of facioscapulohumeral muscular dystrophy gene on chromosome 4. Lancet, 336, 651-653.
5. Wijmenga,C., Hewitt,J.E., Sandkuijl,L.A., Clark,L.N., Wright,T.J., Dauwerse,H.G., Gruter,A.M., Hofker,M.H., Moerer,P., Williamson,R., et al. (1992) Chromosome 4q DNA rearrangements associated with facioscapulohumeral muscular dystrophy. Nat Genet, 2, 26-30.
6. van der Maarel,S.M., Tawil,R., Tapscott,S.J. (2011) Facioscapulohumeral muscular dystrophy and DUX4: breaking the silence. Trends Mol. Med., 17, 252-258.
7. Lemmers,R.J., van der Vliet,P.J., Klooster,R., Sacconi,S., Camano,P., Dauwerse,J.G., Snider,L., Straasheijm,K.R., van Ommen,G.J., Padberg,G.W., et al. (2010) A unifying genetic model for facioscapulohumeral muscular dystrophy. Science, 329, 1650-1653.
8. Snider,L., Geng,L.N., Lemmers,R.J., Kyba,M., Ware,C.B., Nelson,A.M., Tawil,R., Filippova,G.N., van der Maarel,S.M., Tapscott,S.J., Miller,D.G. (2010) Facioscapulohumeral dystrophy: incomplete suppression of a retrotransposed gene. PLoS. Genet., 6, e1001181.
9. Geng,L.N., Yao,Z., Snider,L., Fong,A.P., Cech,J.N., Young,J.M., van der Maarel,S.M., Ruzzo,W.L., Gentleman,R.C., Tawil,R., Tapscott,S.J. (2012) DUX4 activates germline genes, retroelements, and immune mediators: implications for facioscapulohumeral dystrophy. Dev. Cell, 22, 38-51.
10. Kowaljow,V., Marcowycz,A., Ansseau,E., Conde,C.B., Sauvage,S., Matteotti,C., Arias,C., Corona,E.D., Nunez,N.G., Leo,O., et al. (2007) The DUX4 gene at the FSHD1A locus encodes a pro-apoptotic protein. Neuromuscul Disord, 17, 611-623.
11. Wallace,L.M., Garwick,S.E., Mei,W., Belayew,A., Coppee,F., Ladner,K.J., Guttridge,D., Yang,J., Harper,S.Q. (2011) DUX4, a candidate gene for facioscapulohumeral muscular dystrophy, causes p53-dependent myopathy in vivo. Ann. Neurol., 69, 540-552.
12. Gilbert,J.R., Stajich,J.M., Wall,S., Carter,S.C., Qiu,H., Vance,J.M., Stewart,C.S., Speer,M.C., Pufky,J., Yamaoka,L.H., al,e. (1993) Evidence for heterogeneity in facioscapulohumeral muscular dystrophy (FSHD). Am J Hum Genet, 53, 401-408.
13. van Deutekom,J.C., Wijmenga,C., van Tienhoven,E.A., Gruter,A.M., Hewitt,J.E., Padberg,G.W., van Ommen,G.J., Hofker,M.H., Frants,R.R. (1993) FSHD associated DNA rearrangements are due to deletions of integral copies of a 3.2 kb tandemly repeated unit. Hum. Mol. Genet., 2, 2037-2042.
14. Lemmers,R.J., Tawil,R., Petek,L.M., Balog,J., Block,G.J., Santen,G.W., Amell,A.M., van der Vliet,P.J., Almomani,R., Straasheijm,K.R., et al. (2012) Digenic inheritance of an SMCHD1 mutation and an FSHD-permissive D4Z4 allele causes facioscapulohumeral muscular dystrophy type 2. Nat. Genet., 44, 1370-1374.
15. Sacconi,S., Lemmers,R.J., Balog,J., van der Vliet,P.J., Lahaut,P., van Nieuwenhuizen,M.P., Straasheijm,K.R., Debipersad,R.D., Vos-Versteeg,M., Salviati,L., et al. (2013) The FSHD2 gene SMCHD1 is a modifier of disease severity in families affected by FSHD1. Am. J. Hum. Genet., 93, 744-751.
16. Bodega,B., Ramirez,G.D., Grasser,F., Cheli,S., Brunelli,S., Mora,M., Meneveri,R., Marozzi,A., Mueller,S., Battaglioli,E., Ginelli,E. (2009) Remodeling of the chromatin structure of the facioscapulohumeral muscular dystrophy (FSHD) locus and upregulation of FSHD-related gene 1 (FRG1) expression during human myogenic differentiation. BMC. Biol., 7, 41.
17. Cabianca,D.S., Casa,V., Bodega,B., Xynos,A., Ginelli,E., Tanaka,Y., Gabellini,D. (2012) A long ncRNA links copy number variation to a polycomb/trithorax epigenetic switch in FSHD muscular dystrophy. Cell, 149, 819-831.
18. de Greef,J.C., Lemmers,R.J., van Engelen,B.G., Sacconi,S., Venance,S.L., Frants,R.R., Tawil,R., van der Maarel,S.M. (2009) Common epigenetic changes of D4Z4 in contractiondependent and contraction-independent FSHD. Hum. Mutat., 30, 1449-1459.
19. Hartweck,L.M., Anderson,L.J., Lemmers,R.J., Dandapat,A., Toso,E.A., Dalton,J.C., Tawil,R., Day,J.W., van der Maarel,S.M., Kyba,M. (2013) A focal domain of extreme demethylation within D4Z4 in FSHD2. Neurology, 80, 392-399.
20. van Overveld,P.G., Lemmers,R.J., Sandkuijl,L.A., Enthoven,L., Winokur,S.T., Bakels,F., Padberg,G.W., van Ommen,G.J., Frants,R.R., van der Maarel,S.M. (2003) Hypomethylation of D4Z4 in 4q-linked and non-4q-linked facioscapulohumeral muscular dystrophy. Nat. Genet, 35, 315-317.
21. Zeng,W., de Greef,J.C., Chien,R., Kong,X., Gregson,H.C., Winokur,S.T., Pyle,A., Robertson,K.D., Schmiesing,J.A., Ball,R.J., et al. (2009) Specific loss of histone H3 lysine 9 trimethylation and HP1□/cohesin binding at D4Z4 repeats in facioscapulohumeral dystrophy (FSHD). PLoS. Genet, 7, e1000559.
22. Balog,J., Thijssen,P.E., de Greef,J.C., Shah,B., van Engelen,B.G., Yokomori,K., Tapscott,S.J., Tawil,R., van der Maarel,S.M. (2012) Correlation analysis of clinical parameters with epigenetic modifications in the DUX4 promoter in FSHD. Epigenetics., 7, 579-584.
23. Zeng,W., Chen,Y.Y., Newkirk,D.A., Wu,B., Balog,J., Kong,X., Ball,A.R., Jr., Zanotti,S., Tawil,R., Hashimoto,N., et al. (2014) Genetic and Epigenetic Characteristics of FSHDAssociated 4q and 10q D4Z4 that are Distinct from Non-4q/10q D4Z4 Homologs. Hum. Mutat., 35, 998-1010.
24. Snider,L., Asawachaicharn,A., Tyler,A.E., Geng,L.N., Petek,L.M., Maves,L., Miller,D.G., Lemmers,R.J., Winokur,S.T., Tawil,R., et al. (2009) RNA Transcripts, miRNA-sized Fragments, and Proteins Produced from D4Z4 Units: New Candidates for the Pathophysiology of Facioscapulohumeral Dystrophy. Hum. Mol. Genet, 18, 2414-2430.
25. Jones,T.I., Chen,J.C., Rahimov,F., Homma,S., Arashiro,P., Beermann,M.L., King,O.D., Miller,J.B., Kunkel,L.M., Emerson,C.P., Jr., et al. (2012) Facioscapulohumeral muscular dystrophy family studies of DUX4 expression: evidence for disease modifiers and a quantitative model of pathogenesis. Hum. Mol. Genet., 21, 4419-4430.
26. Krom,Y.D., Dumonceaux,J., Mamchaoui,K., den,H.B., Mariot,V., Negroni,E., Geng,L.N., Martin,N., Tawil,R., Tapscott,S.J., et al. (2012) Generation of isogenic D4Z4 contracted and noncontracted immortal muscle cell clones from a mosaic patient: a cellular model for FSHD. Am. J. Pathol., 181, 1387-1401.
27. Tassin,A., Laoudj-Chenivesse,D., Vanderplanck,C., Barro,M., Charron,S., Ansseau,E., Chen,Y.W., Mercier,J., Coppee,F., Belayew,A. (2013) DUX4 expression in FSHD muscle cells: how could such a rare protein cause a myopathy? J. Cell Mol. Med. Vol. 17: 76-89.
28. Asp,P., Blum,R., Vethantham,V., Parisi,F., Micsinai,M., Cheng,J., Bowman,C., Kluger,Y.,Dynlacht,B.D. (2011) Genome-wide remodeling of the epigenetic landscape during myogenic differentiation. Proc. Natl. Acad. Sci. U. S. A, 108, E149-E158.
29. Nozawa,R.S., Nagao,K., Igami,K.T., Shibata,S., Shirai,N., Nozaki,N., Sado,T., Kimura,H., Obuse,C. (2013) Human inactive X chromosome is compacted through a PRC2-independent SMCHD1-HBiX1 pathway. Nat. Struct. Mol. Biol., 20, 566-573.
30. Barth,T.K., Imhof,A. (2010) Fast signals and slow marks: the dynamics of histone modifications. Trends Biochem. Sci., 35, 618-626.
31. Stadler,G., Rahimov,F., King,O.D., Chen,J.C., Robin,J.D., Wagner,K.R., Shay,J.W., Emerson,C.P., Jr., Wright,W.E. (2013) Telomere position effect regulates DUX4 in human facioscapulohumeral muscular dystrophy. Nat. Struct. Mol. Biol., 20, 671-678.
32. Yao,Z., Fong,A.P., Cao,Y., Ruzzo,W.L., Gentleman,R.C., Tapscott,S.J. (2013) Comparison of endogenous and overexpressed MyoD shows enhanced binding of physiologically bound sites. Skelet. Muscle, 3, 8.
33. Thijssen,P.E., Tobi,E.W., Balog,J., Schouten,S.G., Kremer,D., El,B.F., Henneman,P., Putter,H., Eline,S.P., Heijmans,B.T., van der Maarel,S.M. (2013) Chromatin remodeling of human subtelomeres and TERRA promoters upon cellular senescence: commonalities and differences between chromosomes. Epigenetics., 8, 512-521.

## Claims

1. A composition for use in treating facioscapulohumeral muscular dystrophy (FSHD) in a subject, which composition comprises a therapeutically effective amount of a substance which increases the level and/or activity of a protein encoded by a structural maintenance of chromosomes flexible hinge domain containing 1 (*SMCHD1*) gene in cells of the subject, wherein the substance comprises a nucleic acid segment encoding a polypeptide that has an amino acid sequence identity of at least 80%, preferably at least 90%, more preferably at least 95%, most preferably 100% with the SMCHD1 protein of SEQ ID:NO 1.

2. Composition for use according to claim 1, wherein the substance comprises a vector which is suitable for transfecting or transducing a muscle cell or a precursor thereof of the subject and which vector comprises at least one coding region of SMCHD1, preferably as encoded by SEQ ID:NO 2.

3. Composition for use according to claim 2, wherein the vector is an adeno-associated virus vector or a lentivirus vector, more particularly a lentivirus vector carrying the SMCHD1 gene.

4. Composition for use according to claim 1, wherein the substance is SMCHD1 protein of SEQ ID: NO 1, or a protein that has an amino acid sequence identity of at least 80%, preferably at least 90%, more preferably at least 95%, most preferably 100% with the SMCHD1 protein of SEQ ID:NO 1 which protein is capable of binding to chromosome 4q35 D4Z4 repeat array in FSHD cells of the subject.

5. A composition for use in treating FSHD in a subject, which composition comprises a therapeutically effective amount of a substance which increases the level and/or activity of a protein encoded by a SMCHD1 gene in cells of the subject, wherein the substance mediates post-translational modification of SMCHD1 by one or more of sumoylation, ubiquitination and phosphorylation of the SMCHD1 protein;
wherein the composition comprises:
- N106 (N-(4-methoxybenzo[d]thiazol-2-yl)-5-(4-methoxyphenyl)-1,3,4-oxadiazol-2-amine),
- PR-619,
- SENP2 shRNA TRCN0000 004 577; SENP2 shRNA TRCN0000 004 579 or a combination thereof, or
- ubiquitin-like modifier activating enzyme 2 (UBA2) or an amino acid sequence that has an amino acid sequence identity of at least 80%, preferably at least 90%, more preferably at least 95%, most preferably 100% with the UBA2 protein, or a nucleic acid segment encoding said UBA2, which increases a small ubiquitin-like modifier (SUMO)-activating enzyme level.

6. Composition for use according to any of claims 1-5 in treating FSHD type 1 or type 2.

## Patentansprüche

1. Zusammensetzung zur Verwendung bei der Behandlung von fazioskapulohumeraler Muskeldystrophie (FSHD) in einem Subjekt, wobei die Zusammensetzung eine therapeutisch wirksame Menge einer Substanz umfasst, die den Spiegel und/oder die Aktivität eines Proteins, kodiert durch ein strukturelle Aufrechterhaltung der flexiblen Scharnierdomäne von Chromosomen enthaltend 1 (*SMCHD1*) Gen, in Zellen des Subjekts erhöht, wobei die Substanz ein Nukleinsäuresegment umfasst, kodierend ein Polypeptid, das eine Aminosäuresequenzidentität von wenigstens 80 %, vorzugsweise wenigstens 90 %, bevorzugter wenigstens 95 %, am meisten bevorzugt 100 % mit dem SMCHD1-Protein von SEQ ID:NR 1 hat.

2. Zusammensetzung zur Verwendung nach Anspruch 1, wobei die Substanz einen Vektor umfasst, der zum Transfizieren oder Transduzieren einer Muskelzelle oder eines Vorläufers davon des Subjekts geeignet ist und wobei der Vektor wenigstens einen kodierenden Bereich von SMCHD 1 umfasst, vorzugsweise wie kodiert durch SEQ ID:NR 2.

3. Zusammensetzung zur Verwendung nach Anspruch 2, wobei der Vektor ein Adeno-assoziierter Virusvektor oder ein Lentivirusvektor, besonderer ein Lentivirusvektor, tragend das SMCHD 1-Gen, ist.

4. Zusammensetzung zur Verwendung nach Anspruch 1, wobei die Substanz SMCHD 1-Protein von SEQ ID: NR 1, oder ein Protein ist, das eine Aminosäuresequenzidentität von wenigstens 80 %, vorzugsweise wenigstens 90 %, bevorzugter wenigstens 95 %, am meisten bevorzugt 100 % mit dem SMCHD1-Protein von SEQ ID:NR 1 hat, wobei das Protein fähig zum Binden an das Chromosom 4q35 D4Z4-Repeat-Array in FSHD-Zellen des Subjekts ist.

5. Zusammensetzung zur Verwendung bei der Behandlung von FSHD bei einem Subjekt, wobei die Zusammensetzung eine therapeutisch wirksame Menge einer Substanz umfasst, die den Spiegel und/oder die Aktivität eines Proteins, kodiert durch ein SMCHD1-Gen in Zellen des Subjekts, erhöht, wobei die Substanz post-translationale Modifikation von SMCHD1 durch eines oder mehrere von Sumoylierung, Ubiquitinierung oder Phosphorylierung des SMCHD 1-Proteins vermittelt;
wobei die Zusammensetzung umfasst:
- N106 (N-(4-Methoxybenzo[d]thiazol-2-yl)-5-(4-Methoxyphenyl)-1,3,4-oxadiazol-2-amin),
- PR-619,
- SENP2 shRNA TRCN0000 004 577; SENP2 shRNA TRCN0000 004 579 oder eine Kombination davon, oder
- ubiquitinähnlichen Modifikator, aktivierend Enzym 2 (UBA2), oder eine Aminosäuresequenz, die eine Aminosäuresequenzidentität von wenigstens 80 %, vorzugsweise wenigstens 90 %, bevorzugter wenigstens 95 %, am meisten bevorzugt 100 % mit dem UBA2-Protein hat, oder ein Nukleinsäuresegment, kodierend das UBA2, das den Spiegel eines kleinen ubiquitinähnlichen Modifikator (SUMO)-aktivierenden Enzyms erhöht.

6. Zusammensetzung zur Verwendung nach einem der Ansprüche 1 - 5 zur Behandlung von FSHD Typ 1 oder Typ 2.

## Revendications

1. Composition destinée à être utilisée dans le traitement de la dystrophie musculaire facio-scapulo-humérale (FSHD) chez un sujet, laquelle composition comprend une quantité thérapeutiquement efficace d'une substance qui augmente le niveau et/ou l'activité d'une protéine codée par un gène contenant le domaine charnière flexible de maintenance structurale des chromosomes 1 (SMCHD1) dans des cellules du sujet, dans laquelle la substance comprend un segment d'acide nucléique codant un polypeptide qui a une identité de séquence d'acides aminés d'au moins 80 %, de préférence d'au moins 90 %, de préférence encore d'au moins 95 %, de manière particulièrement préférable de 100 % avec la protéine SMCHD1 de SEQ ID: NO 1.

2. Composition destinée à être utilisée selon la revendication 1, dans laquelle la substance comprend un vecteur qui est approprié pour la transfection ou la transduction d'une cellule musculaire ou un précurseur de celle-ci du sujet et lequel vecteur comprend au moins une région codante de SMCHD1, de préférence telle que codée par SEQ ID: NO 2.

3. Composition destinée à être utilisée selon la revendication 2, dans laquelle le vecteur est un vecteur virus adéno-associé ou un vecteur lentivirus, plus particulièrement un vecteur lentivirus portant le gène SMCHD1.

4. Composition destinée à être utilisée selon la revendication 1, dans laquelle la substance est la protéine SMCHD1 de SEQ ID: NO 1, ou une protéine qui a une identité de séquence d'acides aminés d'au moins 80 %, de préférence d'au moins 90 %, de préférence encore d'au moins 95 %, de manière particulièrement préférable de 100 % avec la protéine SMCHD1 de SEQ ID: NO 1, laquelle protéine est capable de se lier à la série de répétitions D4Z4 du chromosome 4q35 dans des cellules FSHD du sujet.

5. Composition destinée à être utilisée dans le traitement de la FSHD chez un sujet, laquelle composition comprend une quantité thérapeutiquement efficace d'une substance qui augmente le niveau et/ou l'activité d'une protéine codée par un gène SMCHD1 dans des cellules du sujet, où la substance assure la médiation de la modification post-traductionnelle de SMCHD1 par une ou plusieurs parmi la sumoylation, l'ubiquitination et la phosphorylation de la protéine SMCHD1;
dans laquelle la composition comprend:
- N106 (N-(4-méthoxybenzo[d]thiazol-2-yl)-5-(4-méthoxy-phényl)-1,3,4-oxadiazol-2-amine),
- PR-619,
- ARNsh de SENP2 TRCN0000 004 577; ARNsh de SENP2 TRCN0000 004 579 ou une combinaison de ceux-ci, ou
- l'enzyme activant le modificateur de type ubiquitine 2 (UBA2) ou une séquence d'acides aminés qui a une identité de séquence d'acides aminés d'au moins 80 %, de préférence d'au moins 90 %, de préférence encore d'au moins 95 %, de manière particulièrement préférable de 100 % avec la protéine UBA2, ou un segment d'acide nucléique codant ladite UBA2, qui augmente le niveau d'une enzyme activant le petit modificateur de type ubiquitine (SUMO).

6. Composition destinée à être utilisée selon l'une quelconque des revendications 1 à 5 dans le traitement de la FSHD de type 1 ou de type 2.
